# EUROPEAN PATENT SPECIFICATION

(11) **EP 1 163 335 B1**
(45) Date of publication and mention of the grant of the patent: **25.05.2005**
(21) Application number: 00911131.1
(22) Date of filing: 22.03.2000
(51) Int. Cl.: C12N 15/12, C12N 15/11, C07K 14/47, C12Q 1/68, G01N 33/53, A61K 39/395, A61K 31/70

(54) **MATERIALS AND METHODS RELATING TO MODULATION OF p66 EXPRESSION**
WERKSTOFFE UND VERFAHREN ZUR MODULIERUNG DER P66 EXPRESSION
MATERIAUX ET PROCEDES RELATIFS A LA MODULATION DE L'EXPRESSION DE p66

(30) Priority: 22.03.1999 GB 9906515
(43) Date of publication of application: 19.12.2001
(73) Proprietor: Congenia S.r.l., 20121 Milan (IT)
(72) Inventor: PELICCI, Pier Giuseppe Istituto Europeo di, 20141 Milan (IT); GIORGIO, Marco Istituto Europeo di Oncologia, 20141 Milan (IT); MIGLIACCIO, Enrica Istituto Europeo di Oncologia, 20141 Milan (IT); LANFRANCONE, Luisa Istituto Europeo di Oncologia, 20141 Milan (IT)
(74) Representative: Cripps, Joanna Elizabeth
(86) International application number: PCT/GB2000/001079
(87) International publication number: WO 2000/056886

(56) References cited:
- WO-A-96/17866
- HARUN R B ET AL: "Characterization of human SHC p66 cDNA and its processed pseudogene mapping to Xq12-q13.1" GENOMICS,US,ACADEMIC PRESS, SAN DIEGO, vol. 42, no. 2, 1 June 1997 (1997-06-01), pages 349-352-352, XP002107843 ISSN: 0888-7543 -& HARUN ET AL.: "shc transforming protein" EMBL DATABASE ACC. NO: Y09847, 1 December 1992 (1992-12-01), XP002142438
- EL-SHEMERLY ET AL: "12-O-Tetradecanoylphorbol-13-acetate activates the Ras/extracellular signal-regulated kinase (ERK) signalling pathway upstream of SOS involving serine phosphorylation of Shc in NIH3T3 cells" THE JOURNAL OF BIOLOGICAL CHEMISTRY, vol. 272, no. 49, 5 December 1997 (1997-12-05), pages 30599-30602, XP002142439
- LESLIE NICK R ET AL: "An activating mutation in the kit receptor abolishes the stroma requirement for growth of ELM erythroleukemia cells, but does not prevent their differentiation in response to erythropoietin." BLOOD, vol. 92, no. 12, 15 December 1998 (1998-12-15), pages 4798-4807, XP000915258 ISSN: 0006-4971
- MIGLIACCIO ET AL.: "Opposite effects of the p52shc/p46shc and p66shc splicing isoforms on the EGF receptor-MAP kinase-fos signalling pathway" THE EMBO JOURNAL, vol. 16, no. 4, 1997, pages 706-716, XP002142441
- RAO ET AL.: "Role of hydroperoxyeicosatetranoic acids in oxidative stress-induced activating protein 1 (AP-1) activity" THE JOURNAL OF BIOLOGICAL CHEMISTRY, vol. 271, no. 44, 1 November 1996 (1996-11-01), pages 27760-27764, XP002142442
- MIGLIACCIO ET AL.: "The p66shc adaptor protein controls oxidative stress response and life span in mamals" NATURE, vol. 402, 18 November 1999 (1999-11-18), pages 309-313, XP002142443

### SEQUENCE LISTING

<110> Cancer Research Ventures Limited
   Pelicci, Pier G
   Giorgio, Marco
   Migliaccio, Enrica
   Lanfrancone, Luisa
<120> Materials and methods relating to modulation of p66
   expression
<130> JEC/BP5846738
<140> PCT/GB00/01079
   <141> 2000-03-22
<150> GB 9906515.3
   <151> 1999-03-22
<160> 2
<170> PatentIn Ver. 2.1
<210> 1
   <211> 3664
   <212> DNA
   <213> Homo sapiens
<400> 1
   atggggcctg aaactgtctg ggtctgagct ggggagcgga agccacttgt ccctctccct 60
   ccccaggact tctgtgactc ctgggccaca gaggtccaac cagggtaagg gcctggggat 120
   accccctgcc tggccccctt gcccaaactg gcaggggggc caggctgggc agcagcccct 180
   ctttcacctc aactatggat ctcctgcccc ccaagcccaa gtacaatcca ctccggaatg 240
   agtctctgtc atcgctggag gaaggggctt ctgggtccac ccccccggag gagctgcctt 300
   ccccatcagc ttcatccctg gggcccatcc tgcctcctct gcctggggac gatagtccca 360
   ctaccctgtg ctccttcttc ccccggatga gcaacctgag gctggccaac ccggctgggg 420
   ggcgcccagg gtctaagggg gagccaggaa gggcagctga tgatggggag gggatcgatg 480
   gggcagccat gccagagtca ggccccctac ccctcctcca ggacatgaac aagctgagtg 540
   gaggcggcgg gcgcaggact cgggtggaag ggggccagct tgggggcgag gagtggaccc 600
   gccacgggag ctttgtcaat aagcccacgc ggggctggct gcatcccaac gacaaagtca 660
   tgggacccgg ggtttcctac ttggttcggt acatgggttg tgtggaggtc ctccagtcaa 720
   tgcgtgccct ggacttcaac acccggactc aggtcaccag ggaggccatc agtctggtgt 780
   gtgaggctgt gccgggtgct aagggggcga caaggaggag aaagccctgt agccgcccgc 840
   tcagctctat cctggggagg agtaacctga aatttgctgg aatgccaatc actctcaccg 900
   tctccaccag cagcctcaac ctcatggccg cagactgcaa acagatcatc gccaaccacc 960
   acatgcaatc tatctcattt gcatccggcg gggatccgga cacagccgag tatgtcgcct 1020
   atgttgccaa agaccctgtg aatcagagag cctgccacat tctggagtgt cccgaagggc 1080
   ttgcccagga tgtcatcagc accattggcc aggccttcga gttgcgcttc aaacaatacc 1140
   tcaggaaccc acccaaactg gtcacccctc atgacaggat ggctggcttt gatggctcag 1200
   catgggatga ggaggaggaa gagccacctg accatcagta ctataatgac ttcccgggga 1260
   aggaaccccc cttggggggg gtggtagaca tgaggcttcg ggaaggagcc gctccagggg 1320
   ctgctcgacc cactgcaccc aatgcccaga cccccagcca cttgggagct acattgcctg 1380
   taggacagcc tgttggggga gatccagaag tccgcaaaca gatgccacct ccaccaccct 1440
   gtccaggcag agagcttttt gatgatccct cctatgtcaa cgtccagaac ctagacaagg 1500
   cccggcaagc agtgggtggt gctgggcccc ccaatcctgc tatcaatggc agtgcacccc 1560
   gggacctgtt tgacatgaag cccttcgaag atgctcttcg ggtgcctcca cctccccagt 1620
   cggtgtccat ggctgagcag ctccgagggg agccctggtt ccatgggaag ctgagccggc 1680
   gggaggctga ggcactgctg cagctcaatg gggacttctt ggtacgggag agcacgacca 1740
   cacctggcca gtatgtgctc actggcttgc agagtgggca gcctaagcat ttgctactgg 1800
   tggaccctga gggtgtggtt cggactaagg atcaccgctt tgaaagtgtc agtcacctta 1860
   tcagctacca catggacaat cacttgccca tcatctctgc gggcagcgaa ctgtgtctac 1920
   agcaacctgt ggagcggaaa ctgtgatctg ccctagcgct ctcttccaga agatgccctc 1980
   caatcctttc caccctattc cctaactctc gggacctcgt ttgggagtgt tctgtgggct 2040
   tggccttgtg tcagagctgg gagtagcatg gactctgggt ttcatatcca gctgagtgag 2100
   agggtttgag tcaaaagcct gggtgagaat cctgcctctc cccaaacatt aatcaccaaa 2160
   gtattaatgt acagagtggc ccctcacctg ggcctttcct gtgccaacct gatgcccctt 2220
   ccccaagaag gtgagtgctt gtcatggaaa atgtcctgtg gtgacaggcc cagtggaaca 2280
   gtcacccttc tgggcaaggg ggaacaaatc acacctctgg gcttcagggt atcccagacc 2340
   cctctcaaca cccgcccccc ccatgtttaa actttgtgcc tttgaccatc tcttaggtct 2400
   aatgatattt tatgcaaaca gttcttggac ccctgaattc ttcaatgaca gggatgccaa 2460
   caccttcttg gcttctggga cctgtgttct tgctgagcac cctctccggt ttgggttggg 2520
   ataacagagg caggagtggc agctgtcccc tctccctggg gatatgcaac ccttagagat 2580
   tgccccagag ccccactccc ggccaggcgg gagatggacc cctcccttgc tcagtgcctc 2640
   ctggccgggg cccctcaccc caaggggtct gtatatacat ttcataaggc ctgccctccc 2700
   atgttgcatg cctatgtact ctgcgccaaa gtgcagccct tcctcctgaa gcctctgccc 2760
   tgcctccctt tctgggaggg cggggtgggg gtgactgaat ttgggcctct tgtacagtta 2820
   actctcccag gtggattttg tggaggtgag aaaaggggca ttgagactat aaagcagtag 2880
   acaatcccca cataccatct gtagagttgg aactgcattc ttttaaagtt ttatatgcat 2940
   atattttagg gctgctagac ttactttcct attttctttt ccattgctta ttcttgagca 3000
   caaaatgata atcaattatt acatttatac atcacctttt tgacttttcc aagccctttt 3060
   acagctcttg gcattttcct cgcctaggcc tgtgaggtaa ctgggatcgc accttttata 3120
   ccagagacct gaggcagatg aaatttattt ccatctagga ctagaaaaac ttgggtctct 3180
   taccgcgaga ctgagaggca gaagtcagcc cgaatgcctg tcagtttcat ggaggggaaa 3240
   cgcaaaacct gcagttcctg agtaccttct acaggcccgg cccagcctag gcccggggtg 3300
   gccacaccac agcaagccgg ccccccctct tttggccttg tggataaggg agagttgacc 3360
   gttttcatcc tggcctcctt ttgctgtttg gatgtttcca cgggtctcac ttataccaaa 3420
   gggaaaactc ttcattaaag tccgtatttc ttctaaaaaa aaaaaaaaaa aaatacattt 3480
   atacatcacc tttttgactt ttccaagccc ttttacagct cttggcattt tcctcgccta 3540
   ggcctgtgag gtaactggga tcgcaccttt tataccagag acctgaggca gatgaaattt 3600
   atttccatct aggactagaa aaacttgggt ctcttaccgc gagactgaga ggcagaagtc 3660
   agcc                                                              3664
<210> 2
   <211> 583
   <212> PRT
   <213> Homo sapiens
<400> 2
   Met Asp Leu Leu Pro Pro Lys Pro Lys Tyr Asn Pro Leu Arg Asn Glu
   1               5                   10                  15
   Ser Leu Ser Ser Leu Glu Glu Gly Ala Ser Gly Ser Thr Pro Pro Glu
               20                  25                  30
   Glu Leu Pro Ser Pro Ser Ala Ser Ser Leu Gly Pro Ile Leu Pro Pro
           35                  40                  45
   Leu Pro Gly Asp Asp Ser Pro Thr Thr Leu Cys Ser Phe Phe Pro Arg
       50                  55                  60
   Met Ser Asn Leu Arg Leu Ala Asn Pro Ala Gly Gly Arg Pro Gly Ser
   65                  70                  75                  80
   Lys Gly Glu Pro Gly Arg Ala Ala Asp Asp Gly Glu Gly Ile Asp Gly
                   85                  90                  95
   Ala Ala Met Pro Glu Ser Gly Pro Leu Pro Leu Leu Gln Asp Met Asn
               100                 105                 110
   Lys Leu Ser Gly Gly Gly Gly Arg Arg Thr Arg Val Glu Gly Gly Gln
           115                 120                 125
   Leu Gly Gly Glu Glu Trp Thr Arg His Gly Ser Phe Val Asn Lys Pro
       130                 135                 140
   Thr Arg Gly Trp Leu His Pro Asn Asp Lys Val Met Gly Pro Gly Val
   145                 150                 155                 160
   Ser Tyr Leu Val Arg Tyr Met Gly Cys Val Glu Val Leu Gln Ser Met
                   165                 170                 175
   Arg Ala Leu Asp Phe Asn Thr Arg Thr Gln Val Thr Arg Glu Ala Ile
               180                 185                 190
   Ser Leu Val Cys Glu Ala Val Pro Gly Ala Lys Gly Ala Thr Arg Arg
   195 200 205
   Arg Lys Pro Cys Ser Arg Pro Leu Ser Ser Ile Leu Gly Arg Ser Asn
   210 215 220
   Leu Lys Phe Ala Gly Met Pro Ile Thr Leu Thr Val Ser Thr Ser Ser
   225 230 235 240
   Leu Asn Leu Met Ala Ala Asp Cys Lys Gln Ile Ile Ala Asn His His
   245 250 255
   Met Gln Ser Ile Ser Phe Ala Ser Gly Gly Asp Pro Asp Thr Ala Glu
   260 265 270
   Tyr Val Ala Tyr Val Ala Lys Asp Pro Val Asn Gln Arg Ala Cys His
   275 280 285
   Ile Leu Glu Cys Pro Glu Gly Leu Ala Gln Asp Val Ile Ser Thr Ile
   290 295 300
   Gly Gln Ala Phe Glu Leu Arg Phe Lys Gln Tyr Leu Arg Asn Pro Pro
   305 310 315 320
   Lys Leu Val Thr Pro His Asp Arg Met Ala Gly Phe Asp Gly Ser Ala
   325 330 335
   Trp Asp Glu Glu Glu Glu Glu Pro Pro Asp His Gln Tyr Tyr Asn Asp
   340 345 350
   Phe Pro Gly Lys Glu Pro Pro Leu Gly Gly Val Val Asp Met Arg Leu
   355 360 365
   Arg Glu Gly Ala Ala Pro Gly Ala Ala Arg Pro Thr Ala Pro Asn Ala
   370 375 380
   Gln Thr Pro Ser His Leu Gly Ala Thr Leu Pro Val Gly Gln Pro Val
   385 390 395 400
   Gly Gly Asp Pro Glu Val Arg Lys Gln Met Pro Pro Pro Pro Pro Cys
   405 410 415
   Pro Gly Arg Glu Leu Phe Asp Asp Pro Ser Tyr Val Asn Val Gln Asn
   420 425 430
   Leu Asp Lys Ala Arg Gln Ala Val Gly Gly Ala Gly Pro Pro Asn Pro
           435                 440                 445
   Ala Ile Asn Gly Ser Ala Pro Arg Asp Leu Phe Asp Met Lys Pro Phe
       450                 455                 460
   Glu Asp Ala Leu Arg Val Pro Pro Pro Pro Gln Ser Val Ser Met Ala
   465                 470                 475                 480
   Glu Gln Leu Arg Gly Glu Pro Trp Phe His Gly Lys Leu Ser Arg Arg
                   485                 490                 495
   Glu Ala Glu Ala Leu Leu Gln Leu Asn Gly Asp Phe Leu Val Arg Glu
               500                 505                 510
   Ser Thr Thr Thr Pro Gly Gln Tyr Val Leu Thr Gly Leu Gln Ser Gly
           515                 520                 525
   Gln Pro Lys His Leu Leu Leu Val Asp Pro Glu Gly Val Val Arg Thr
       530                 535                 540
   Lys Asp His Arg Phe Glu Ser Val Ser His Leu Ile Ser Tyr His Met
   545                 550                 555                 560
   Asp Asn His Leu Pro Ile Ile Ser Ala Gly Ser Glu Leu Cys Leu Gln
                   565                 570                 575
   Gln Pro Val Glu Arg Lys Leu
               580

## Description

### Field of the Invention

The present invention relates to materials and methods concerned with the effects of p66 expression. Particularly, but not exclusively, the present invention provides materials and methods relating to observations that p66, and more particularly p66 Shc isoform, is part of a signal transduction pathway that regulates stress response, response to oncogenic signals and lifespan in mammals.

### Background of the Invention

The genes that are responsible for the phenomenon of aging in mammals are unknown. Current theories postulate that aging is the consequence of mutations which do not affect fitness of adult individuals, and which have deleterious effects later in life. Circumstantial evidence suggest genes involved in the control of the oxidative stress response are candidate "aging genes". Indeed, accumulation of oxidative damage correlates with aging.

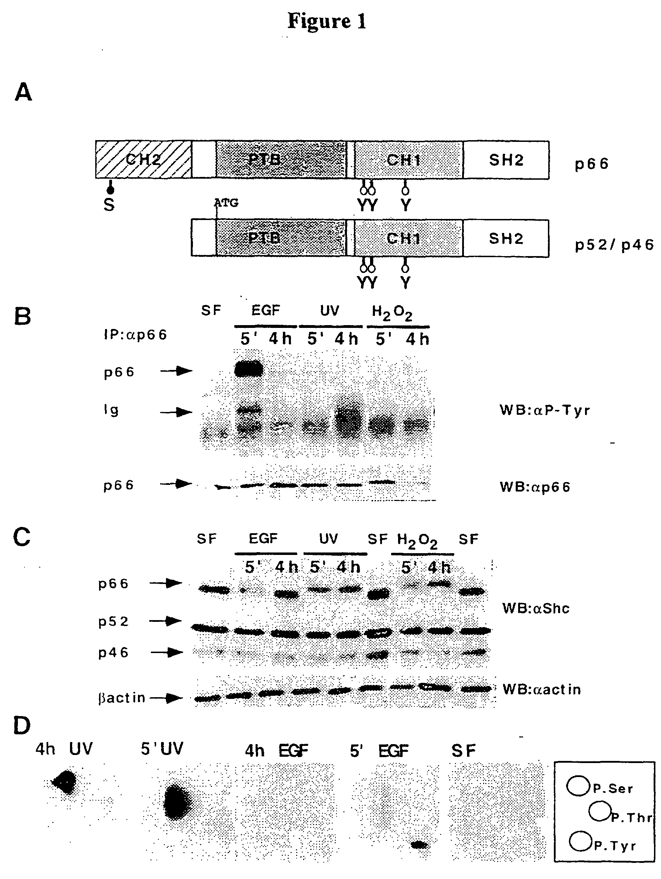

The mammalian SHC locus encodes three isoforms: p52, p46 and p66. They differ by the presence of N-terminal sequences of variable length and share a C-terminal SH2 domain, a central collagen-homology domain (CH1), rich in proline/glycine residues, and an N-terminal phosphotyrosine-binding domain (PTB). The 110 amino acid N-terminal region unique to p66 is also rich in glycine and proline residues (CH2) (Fig.1A). Therefore, p66^{shc} is a splice variant of p52^{shc}/p46^{shc} (Migliaccio E. et al Embo J. 16, 706-716 (1997), a cytoplasmic signal transducer involved in the transmission of mitogenic signals from tyrosine kinases to Ras (Pelicci G. et al Cell 70, 93-104 (1992)). The p52/46 Shc isoforms are involved in the cytoplasmic propagation of mitogenic signals from activated receptors to Ras (Bonfini L. et al Tibs 21, 257-261; 1996). They are rapidly phosphorylated on tyrosine after ligand stimulation of receptors and, upon phosphorylation, form stable complexes with activated receptors and Grb2, an adaptor protein for the Ras guanine nucleotide exchange factor SOS (Migliaccio E. et al Embo J. 16, 706-716 (1997); Pelicci G. et al Cell 70, 93-104 (1992); Rozakis-Adcock, M. et al Nature 360, 689-692 (1992)). These complexes induce Ras activation, as measured by increased RasGTP formation, Mitogen Activated Protein Kinase (MAPK) activity and FOS activation in cultured cells overexpressing p52^{shc}/p46^{shc} (Migliaccio, E. et al Embo J. 16, 706-716 (1997); Pronk, G. et al Mol.Cell. Biol. 14, 1575-1581 (1994); Lanfrancone, L. et al Oncogene 10, 907-917 (1995)). Likewise, p66^{shc} becomes tyrosine-phosphorylated upon receptor activation and forms stable complexes with activated receptors and Grb2. However, it inhibits c-fos promoter activation and does not affect MAPK activity, thereby suggesting that p66^{shc} acts in a distinct intracellular signalling pathway (Migliaccio E. et al Embo J. 16, 706-716 (1997)).

c-fos is transcriptionally activated in response to a large variety of adverse agents (environmental stress), such as DNA-damaging agents (e.g. ultraviolet radiation, UV) or agents that induce oxidative damage (e.g. hydrogen peroxide, H₂0₂) (Schreiber, M. et al Embo J. 14, 5338-5349 (1995); Sen, C. et al FASEB J. 10, 709-720 (1996)).

It is postulated that the major causal factor of aging is the accumulation of oxidative damage as an organism ages (Martin, G. M. et al Nature Genetics 13, 25-34 (1996); Johnson, F.B. et al Cell 96, 291-302 (1999); Lithgow G. J. et al Science 273, 80 (1996)). Indeed, transgenic flies that overexpress antioxidative enzymes have greater longevity (Orr W. C. et al Science 263, 1128-1130 (1994)); restriction of caloric intake lowers steady state levels of oxidative stress and damage and extends the maximum life span in mammals (Sohal R.S. et al Science 273, 59-63 (1996)). However, the genes that determine lifespan in mammals are not known. Among currently accepted evolutionary theories, it is postulated that aging is a post-reproductive process that has escaped the force of natural selection and that evolved through selection of alleles with early life benefits combined with pleiotropically harmful effects later in life. The postulated genes, since actively selected, are, therefore thought to regulate fundamental cellular processes, common to different species.

### Summary of the Invention

The present inventors have determined that p66 is a pivotal gene in the regulation of the cellular responses to environmental and oncogenic stresses and that it is involved in the process of aging and in tumour suppression. p66 provides the first genetic information on the theory of aging. Mechanistically, p66 exerts its functions downstream to stress-activated serine kinases and upstream to p53-p21.

The present inventors have determined that targeted mutations of the mouse p66^{shc} gene induces stress resistance and prolongs survival. The present inventors disclose herein that i) p66^{shc} is serine phosphorylated upon UV treatment or oxidative damage; ii) the serine-phosphorylation of p66 by oxidative signals is mediated by Erk1 and p38, as shown both in vivo and in vitro; iii) ablation of p66^{shc} expression by homologous recombination enhances resistance to oxidative damage both *in vitro* and *in vivo;* iv) a serine-phosphorylation defective mutant of p66^{shc} is unable to restore a normal stress response in p66^{shc} targeted cells; v) mice carrying the p66^{shc} targeted mutation have prolonged lifespan.

The present inventors disclose herein that i) p16, p53 and p21 activation is lost in p66-/- cells upon H₂O₂ or UV treatment or RASV12 expression; ii) the oncogenic RASV12 is unable to induce cell senescence into p66-/- mouse embryo fibroblasts (MEFs) and, on the contrary, it transforms p66-/- cells; iii) p66-/- MEFs over-expressing RASV12 show a transformed, spindle-shaped morphology, are capable of forming foci at confluency and colonies in semisolid media; iv) p16 and p53 are unable to induce growth proliferation of p66-/- cells.

Thus, the present inventors show herein that p66 itself is activated by serine phosphorylation by stress activated kinases and signals to p16-p19-p53-p21 and that functionally, the p66 signalling pathway regulates tumour supression and lifespan.

Therefore, at its most general, the present invention provides materials and methods associated with the modulation of p66^{shc} gene expression and its involvement in a signal transduction pathway that is activated by environmental stresses and oncogenic mutations.

The present invention provides a method of disrupting the p66^{shc} signal transduction pathway in a cell, said method comprising the step of contacting said cell with a nucleic acid molecule capable of hybridizing to nucleic acid encoding p66^{shc} thereby preventing p66^{shc} expression.

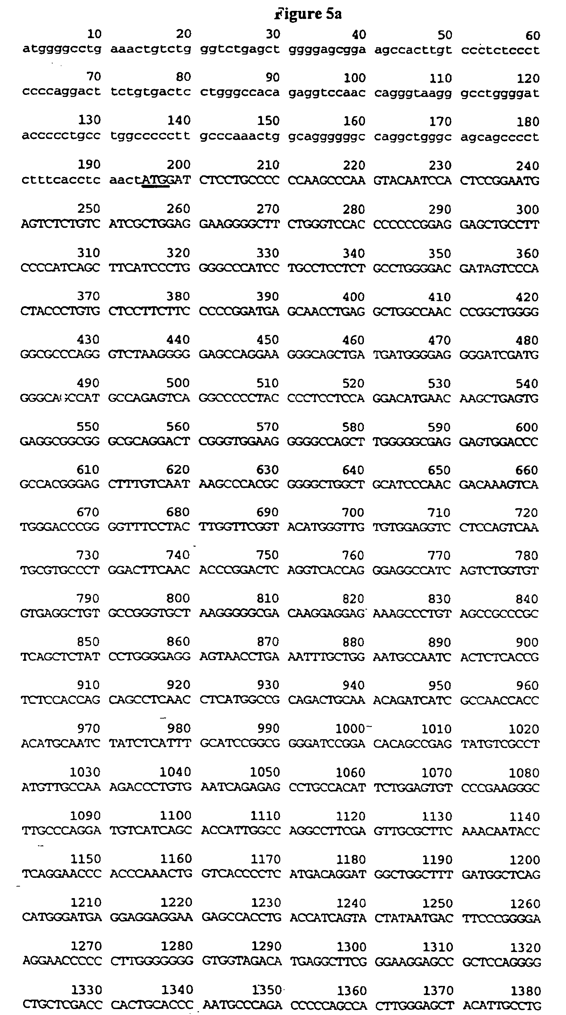

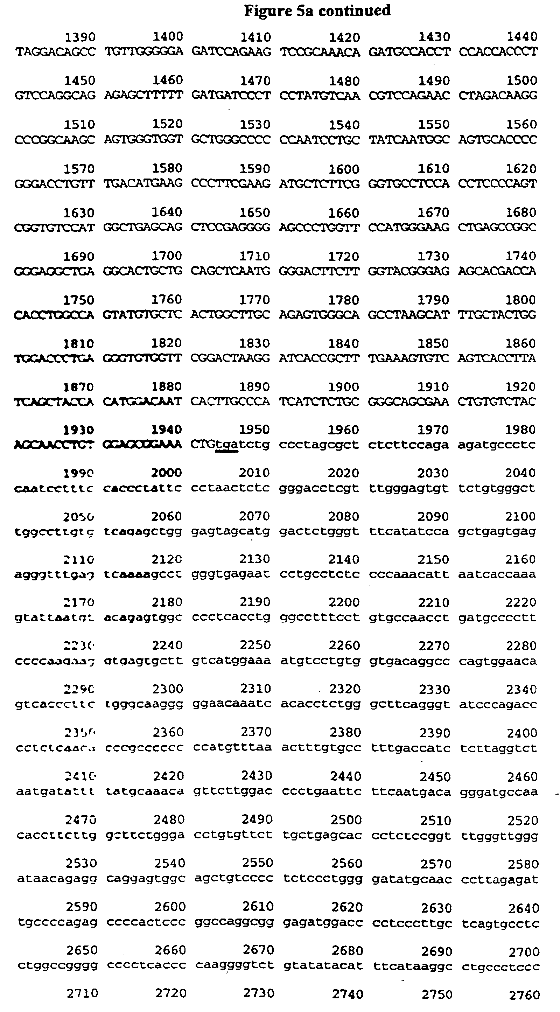

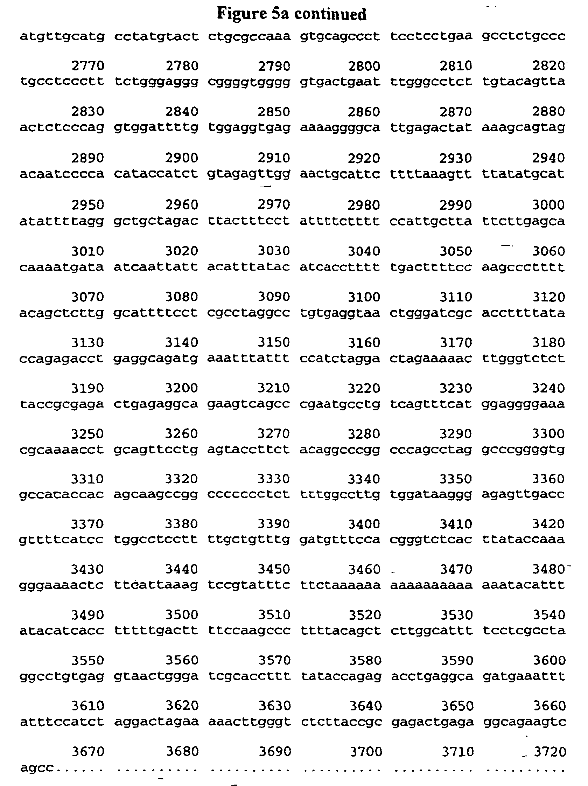

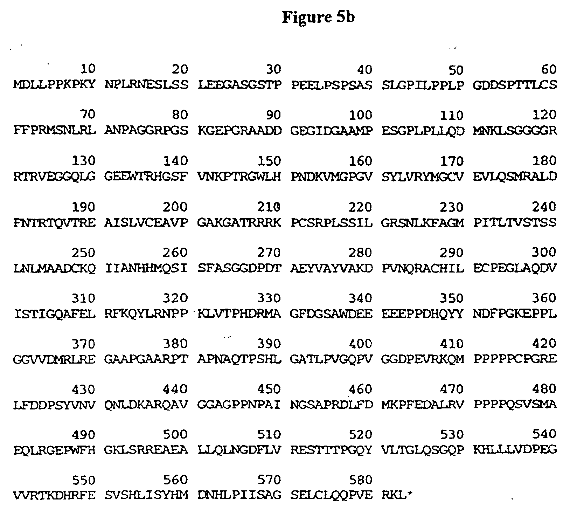

In a first aspect of the present invention there is also provided a nucleic acid molecule comprising a p66^{shc} coding sequence, the coding sequence consisting of the coding sequence of Figure 5 incorporating a mutation such that, is the protein encoded by the coding sequence residue 536 is absent or replaced by a different amino acid residue. Preferably, the serine residue is replaced by a different amino acid residue, for example S36 is replaced by alanine (p66^{shc}S36A).

The present invention further provides a polypeptide encoded for by the nucleic acid molecule of the present invention as disclosed above.

Generally, nucleic acid according to the present invention is provided as an isolate, in isolated and/or purified form, or free or substantially free of material with which it is naturally associated, such as free or substantially free of nucleic acid flanking the gene in the human genome, except possibly one or more regulatory sequence(s) for expression. Nucleic acid may be wholly or partially synthetic and may include genomic DNA, cDNA or RNA. Where nucleic acid according to the invention includes RNA, reference to the sequence shown should be construed as reference to the RNA equivalent, with U substituted for T.

Nucleic acid sequences encoding all or part of the p66^{shc} gene and/or its regulatory elements can be readily prepared by the skilled person using the information and references contained herein and techniques known in the art (for example, see Sambrook, Fritsch and Maniatis, "Molecular Cloning, A Laboratory Manual, Cold Spring Harbor Laboratory Press, 1989, and Ausubel et al, Short Protocols in Molecular Biology, John Wiley and Sons, 1992). These techniques include (i) the use of the polymerase chain reaction (PCR) to amplify samples of such nucleic acid, e.g. from genomic sources, (ii) chemical synthesis, or (iii) preparing cDNA sequences. Modifications to the p66^{shc} sequences can be made, e.g. using site directed mutagenesis, to lead to the expression of modified p66^{shc} polypeptide or to take account of codon preference in the host cells used to express the nucleic acid.

In order to obtain expression of the p66^{shc} nucleic acid sequences, the sequences can be incorporated in a vector having control sequences operably linked to the p66^{shc} nucleic acid to control its expression. The vectors may include other sequences such as promoters or enhancers to drive the expression of the inserted nucleic acid, nucleic acid sequences so that the p66^{shc} polypeptide is produced as a fusion and/or nucleic acid encoding secretion signals so that the polypeptide produced in the host cell is secreted from the cell. p66^{shc} polypeptide can then be obtained by transforming the vectors into host cells in which the vector is functional, culturing the host cells so that the p66^{shc} polypeptide is produced and recovering the p66^{shc} polypeptide from the host cells or the surrounding medium. Prokaryotic and eukaryotic cells are used for this purpose in the art, including strains of E. coli, yeast, and eukaryotic cells such as COS or CHO cells. The choice of host cell can be used to control the properties of the p66^{shc} polypeptide expressed in those cells, e.g. controlling where the polypeptide is deposited in the host cells or affecting properties such as its glycosylation.

PCR techniques for the amplification of nucleic acid are described in US Patent No. 4,683,195. In general, such techniques require that sequence information from the ends of the target sequence is known to allow suitable forward and reverse oligonucleotide primers to be designed to be identical or similar to the polynucleotide sequence that is the target for the amplification. PCR comprises steps of denaturation of template nucleic acid (if double-stranded), annealing of primer to target, and polymerisation. The nucleic acid probed or used as template in the amplification reaction may be genomic DNA, cDNA or RNA. PCR can be used to amplify specific sequences from genomic DNA, specific RNA sequences and cDNA transcribed from mRNA, bacteriophage or plasmid sequences. The p66^{shc} nucleic acid sequences provided herein readily allow the skilled person to design PCR primers. References for the general use of PCR techniques include Mullis et al, Cold Spring Harbor Symp. Quant. Biol., 51:263, (1987), Ehrlich (ed), PCR technology, Stockton Press, NY, 1989, Ehrlich et al, Science, 252:1643-1650, (1991), "PCR protocols; A Guide to Methods and Applications", Eds. Innis et al, Academic Press, New York, (1990).

An oligonucleotide for use in nucleic acid amplification may have about 10 or fewer codons (e.g. 6, 7 or 8), i.e. be about 30 or fewer nucleotides in length (e.g. 18, 21 or 24). Generally specific primers are upwards of 14 nucleotides in length, but not more than 18-20. Those skilled in the art are well versed in the design of primers for use processes such as PCR.

A convenient way of producing a polypeptide according to the present invention is to express nucleic acid encoding it, by use of the nucleic acid in an expression system. The use of expression system has reached an advanced degree of sophistication today.

Accordingly, the present invention also encompasses a method of making a polypeptide (as disclosed), the method including expression from nucleic acid encoding the polypeptide (generally nucleic acid according to the invention). This may conveniently be achieved by growing a host cell in culture, containing such a vector, under appropriate conditions which cause or allow expression of the polypeptide. Polypeptides may also be expressed in in vitro systems, such as reticulocyte lysate.

Systems for cloning and expression of a polypeptide in a variety of different host cells are well known. Suitable host cells include bacteria, eukaryotic cells such as mammalian and yeast, and baculovirus systems. mammalian cell lines available in the art for expression of a heterologous polypeptide include Chinese hamster ovary cells, HeLa cells, baby hamster kidney cells, COS cells and many others. A common, preferred bacterial host is E. coli.

Suitable vectors can be chosen or constructed, containing appropriate regulatory sequences, including promoter sequences, terminator fragments, polyadenylation sequences, enhancer sequences, marker genes and other sequences as appropriate. Vectors may be plasmids, viral e.g. 'phage, or phagemid, as appropriate. For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Ausubel et al. eds., John Wiley & Sons, 1992.

Thus, the present invention further provides a host cell containing a vector as disclosed herein. The nucleic acid of the invention may be integrated into the genome (e.g. chromosome) of the host cell. Integration may be promoted by inclusion of sequences which promote recombination with the genome, in accordance with standard techniques. The nucleic acid may be on an extra-chromosomal vector within the cell.

An antisease oligonucleotide capable of specifically hybridizing to p66^{shc} nucleic acid may be used in the preparation of a medicament for increasing the cellular resistance to oxidative stress.

The present invention also provides in vitro methods of increasing resistance in cells to oxidative stress. Such oxidative stress may be as a result of external, e.g. environmental, factors such as UV, X-rays heat shock, osmotic shock, oxidative stress (singlet oxygen, H₂O₂, hydroxylradicals, inflammatory cytokines). or it may be as a result of internal factors resulting in necrosis of cells as occurs in some disease states.

A method of increasing resistance to oxidative stress comprises disrupting a p66^{shc} signalling pathway. The pathway may be disrupted at any stage during the signalling process, for example, the p66^{shc} polypeptide may be mutated such that the serine residue is absent or replaced by a different amino acid residue, e.g. alanine such that the resulting polypeptide cannot be serine phosphorylated; the ability of molecules such as p38 or MAPK to phosphorylate p66^{shc} may be disrupted by, for example, dominant negative kinases or specific inhibitors; and , most preferably, the expression of p66^{shc} may be disrupted thereby down regulating p66^{shc} polypeptide in the cell. Further,as p53 and p16 are not biologically active in p66-/- cells, any dominant negative p66 molecules may be used to block p16 and p53 function.

The disruption of p66^{shc} gene expression may be obtained in various ways. Antisense oligonucleotide sequences based on the p66^{shc} sequence may be designed to hybridise to the complementary sequence of nucleic acid, pre-mRNA, or mature mRNA, interfering with the production of polypeptide encoded by a given DNA sequence (e.g. either native p66^{shc} polypeptide or a mutant form thereof), so that its expression is reduced or prevented altogether. In addition to the p66^{shc} coding sequence, antisense techniques can be used to target the control sequences of the p66^{shc} gene, e.g. in the 5' flanking sequence of the p66^{shc} coding sequence, whereby the antisense oligonucleotides can interfere with the p66^{shc} control sequences. The construction of antisense sequences and their use is described in Peyman and Ulman, Chemical Reviews, 90:543-584, (1990), Crooke, Ann. Rev. Pharmacol. Toxical., 32:329-376, (1992), and Zamecnik and Stephenson, P.N.A.S., 75:280-284, (1974).

A method of screening for compounds capable of modulating a p66^{shc} signalling pathway may comprise contacting a candidate compound with a p66^{shc} expression system as described above; determining the amount of a component of the signalling pathway; and comparing said amount of the component with the amount of the component in the absence of said candidate compound.

Preferably, the expression system comprises a nucleic acid vector having a p66^{shc} coding sequence or fragment thereof inserted therein. Suitable vectors can be chosen or constructed, containing appropriate regulatory sequences, including promoter sequences, termination fragments, polyadenylation sequences enhancer sequences, marker genes and other sequences as appropriate. For further details see, for example, Molecular cloning: A laboratory Manual: 2nd edition, Sambrook et al, 1989, Cold Spring Harbor Laboratory Press. Procedures for introducing nucleic acid into cells depends on the host cell used, but are well known.

Thus the expression system may also comprises a host cell containing a p66^{shc} coding sequence or a vector as disclosed above. Most preferably, the expression system comprises a cell derived from a cell line, such as mouse embryo fibroblasts, known to express p66^{shc}.

The p66^{shc} signalling pathway may be modulated, e.g. disrupted, at any stage during the signalling pathway such that production of active p66^{shc} is prevented. Examples of such modulation may include directly preventing expression of p66^{shc} by blocking factors involved in the transcription of genes. For example, antisense primers may be used to bind to the p66^{shc} gene thereby preventing transcription factors binding to regulatory agents required for promoting transcription. Alternatively, the coding sequence for p66^{shc} may be targeted so as to introduce mutations which prevent the expression of p66^{shc} without disrupting expression of associated proteins such as P52^{shc} and p46^{shc.} Preferably, the mutation disrupts the exon encoding the p66 CH2 region. Antisense probes may also be used for binding DNA or mRNA encoding p66^{shc} such that its translation is prevented. Alternatively, antibodies specific for p66^{shc} (e.g. anti-CH2 antibodies) may be used to specifically bind to the expressed protein such that subsequent binding of p66^{shc} to other proteins, e.g. receptors, is prevented.

Further, inhibition of the p66 function can be obtained by inhibiting the phosphorylation of the p66 CH2 region induced by Erk 1 or p38 stress-kinases. To this end, library of compounds can be screened to identify those which are able to inhibit the in vitro phophorylation of the GST-CH2 region by recombinant Erkl. Examples of such compounds include MAPK (mitogen activated protein kinase), any serine/threonine kinase, and p38.

Antibodies may also be used for affecting or modulating the activity or function of p66^{Shc}. Thus, a further and important use of the p66^{Shc} polypeptide is in the raising of antibodies that have the property of specifically binding to the p66^{Shc} polypeptide, fragments or active portions thereof.

The production of monoclonal antibodies is well established in the art. Monoclonal antibodies can be subjected to the techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP-A-184187, GB-A-2188638 or EP-A-239400. A hybridoma producing a monoclonal antibody may be subject to genetic mutation or other changes, which may or may not alter the binding specificity of antibodies produced.

An antibody able to bind specifically to the p66^{Shc} polypeptide may be specific in the sense of being able to distinguish between the polypeptide it is able to bind and other human polypeptides for which it has no or substantially no binding affinity (e.g. a binding affinity of about 1000x worse). Specific antibodies bind an epitope on the molecule which is either not present or is not accessible on other molecules. Antibodies according to the present invention are preferably specific for the wild-type p66^{Shc} polypeptide such that they disrupt its function.

Preferred antibodies are isolated, in the sense of being free from contaminants such as antibodies able to bind other polypeptides and/or free of serum components. Monoclonal antibodies are preferred for some purposes, though polyclonal antibodies are within the scope of the present invention.

Antibodies may be obtained using techniques which are standard in the art. Methods of producing antibodies include immunising a mammal (e.g. mouse, rat, rabbit, horse, goat, sheep or monkey) with the protein or a fragment thereof. Antibodies may be obtained from immunised animals using any of a variety of techniques known in the art, and screened, preferably using binding of antibody to antigen of interest. For instance, Western blotting techniques or immunoprecipitation may be used (Armitage et al, Nature, 357:80-82, 1992). Isolation of antibodies and/or antibody-producing cells from an animal may be accompanied by a step of sacrificing the animal.

As an alternative or supplement to immunising a mammal with a peptide, an antibody specific for a protein may be obtained from a recombinantly produced library of expressed immunoglobulin variable domains, e.g. using lambda bacteriophage or filamentous bacteriophage which display functional immunoglobulin binding domains on their surfaces; for instance see WO92/01047. The library may be naive, that is constructed from sequences obtained from an organism which has not been immunised with any of the proteins (or fragments), or may be one constructed using sequences obtained from an organism which has been exposed to the antigen of interest.

Antibodies according to the present invention may be modified in a number of ways. Indeed the term "antibody" should be construed as covering any binding substance having a binding domain with the required specificity. Thus the invention covers antibody fragments, derivatives, functional equivalents and homologues of antibodies, including synthetic molecules and molecules whose shape mimics that of an antibody enabling it to bind an antigen or epitope.

Example antibody fragments, capable of binding an antigen or other binding partner are the Fab fragment consisting of the VL, VH, Cl and CH1 domains; the Fd fragment consisting of the VH and CH1 domains; the Fv fragment consisting of the VL and VH domains of a single arm of an antibody; the dAb fragment which consists of a VH domain; isolated CDR regions and F(ab')2 fragments, a bivalent fragment including two Fab fragments linked by a disulphide bridge at the hinge region. Single chain Fv fragments are also included.

It is well known that pharmaceutical research leading to the identification of a new drug may involve screening of very large numbers of candidate substances, both before and even after a lead compound has been found. This is one factor which makes pharmaceutical research very expensive and time consuming. Means for assisting in the screening process can have considerable commercial importance and utility. Such means for screening for substances potentially useful in increasing resistance to oxidative stress and thus extending cellular longevity, is provided by the present invention.

A method for screening for a substance which modulates (disrupts) activity of the p66^{shc} polypeptide may include contacting one or more test substances with the polypeptide in a suitable reaction medium, testing the activity of the treated polypeptide and comparing that activity with the activity of the polypeptide in comparable reaction medium untreated with the test substance or substances. Conveniently, an enzyme activity assay may be used to determine any disruption in the activity of p66^{Shc}.

Combinatorial library technology provides an efficient way of testing a potentially large number of different substances for ability to disrupt or delete activity of p66^{shc}. Such libraries and their use are known in the art. The use of peptide libraries is preferred.

Further, assays for determining p66 inhibitors may include usage of wildtype cells (both established cell lines or primary cells capable of expressing p66^{shc}, e.g. MEFs, p66-/- MEFS or MEFs overexpressing p66 (through the usage of a p66^{shc} expressing vector).

Comparative responses to be determined may include response to stress factors, e.g. UV or H₂O₂; inhibition of RASV12 (or any other oncogene) -induced senescence in primary fibroblasts; inhibition of p53 or p19 or p16 or p21 function (as measured by transcriptional assays, or stability assays, or nucleus-cytoplasmic export assays); or inhibition of p66 phosphorylation induced by RASV12 or by oxidative stress signals.

Following identification of a substance or compound which disrupts p66^{shc} or a step in the p66^{shc} signalling pathway, the substance or compound may be investigated further. Furthermore, it may be manufactured and/or used in the preparation, i.e manufacture or formulation, of a composition such as a medicament, pharmaceutical composition or drug. These may be administered to individuals. Oxidative stress has been implicated in the generation of many human diseases. Thus, medicaments, pharmaceutical compositions or drugs comprising an inhibitor of p66 function, or substances or compounds which disrupt p66^{shc} or a step in the p66^{shc} pathway may be used in the treatment of diseases such as arteriosclerosis; ischemic heart disease, Parkinson, Alzheimer, vascular complications of diabetes, emphysema and other lung dieases, myocardial infarction, stroke, premature aging, cell senescence, skin diseases and cancers.

The present invention also provides an in vitro method of increasing cellular resistance to oxidative stress comprising deleting or disrupting the gene encoding p66^{shc} from the cellular genome. Such a method may include processes wherein a nucleic acid vector comprises a nucleic acid sequence capable of being incorporated into the genome of the cell and disrupting the expression of p66^{shc}.

Vectors such as viral vectors have been used in the prior art to introduce genes into a wide variety of different target cells. Typically the vectors are exposed to the target cells so that transfection can take place in a sufficient proportion of the cells to provide a useful therapeutic or prophylactic effect from the expression of the desired polypeptide. The transfected nucleic acid may be permanently incorporated into the genome of each of the targeted tumour cells, providing long lasting effect, or alternatively the treatment may have to be repeated periodically.

A variety of vectors, both viral vectors and plasmid vectors, are known in the art, see US Patent No. 5,252,479 and WO 93/07282. In particular, a number of viruses have been used as gene transfer vectors, including papovaviruses, such as SV40, vaccinia virus, herpes viruses, including HSV and EBV, and retroviruses. Many gene therapy protocols in the prior art have used disabled murine retroviruses.

As an alternative to the use of viral vectors other known methods of introducing nucleic acid into cells includes electroporation, calcium phosphate co-precipitation, mechanical techniques such as microinjection, transfer mediated by liposomes and direct DNA uptake and receptor-mediated DNA transfer.

As mentioned above, the present inventors have also determined that p66 expression is indispensible for the capacity of p53 to induce apoptosis (following, for example, oxidative stress) or senescence (following for example, Ras expression). Thus, agonists of p66 can be envisioned as putative tumour suppressors. In fact, any molecule that would prevent p66 de-phosphorylation or kinases that induce p66 phosphorylation are potential devices for tumour treatment.

Thus, in a further aspect of the present invention, there is provided the use of a vector comprising nuclein acid encoding p66^{shc} polypeptide in the preparation of a medicament for increasing resistance to tumour formation in a tissue by increasing expression of P66^{shc}. The results disclosed herein indicate that increased levels of P66^{shc} reduce susceptibility to carcinogenesis.

The vector is capable of incorporating said nucleic acid into the genome of cells of the tissue.

For example, nucleic acid encoding authentic biologically active p66^{shc} polypeptide could be used in a method of gene therapy, to treat a patient who is unable to synthesize the active polypeptide or unable to synthesize it at the normal level, thereby providing the effect provided by wild-type p66^{shc} and suppressing the susceptibility to tumour formation.

Aspects and embodiments of the invention will now be illustrated, by way of example, with reference to the accompanying figures. Further aspects of the invention will be apparent to those skilled in the art.

### Brief Description of the Drawings

In the figures

**Figure 1** shows serine phosphoryation of p66^{shc} by UV or H₂O₂ treatment. Figure 1A: Modular organisation of p66^{shc}; Y: Y239, Y340 and Y317, the major Shc tyrosine phosphoryation sites. The alternative initiation codon (ATG) of p46^{shc} is indicated. Figure 1B: Anti-phosphotyrosine Western blotting of anti-p66 (αCH2) immunoprecipitates from lystates of serum starved MEFs (SF) or Mouse Embryo Fibroblasts (MEFs) treated with EGF, UV or H₂O₂ for 5 min or 4 hrs, as indicated. The same blot was reprobed with anti-p66^{shc} antibodies (α-CH2). The p66^{shc} polypeptides are arrowed. Immunoglobulin cross-reactive polypeptides are also indicated (Ig). Figure 1C: Western blotting analysis of p66^{shc} expression of serum starved MEFs (SF) or MEFs treated with EGF, UV or H₂O₂ for 5 min or 4 hrs as indicated. The same blot was reprobed with anti-actin antibodies. Figure 1D: Phosphoaminoacid analysis of p66^{shc}. Serum-starved MEFs (SF) were labelled with 1mCi/ml [³²P] orthophosphate for 4 hr and unstimulated (SF) or cells stimulated with EGF, UV or H₂O₂ for 5 min or 4 hr were lysed and immunoprecipitates were resolved by SDS-PAGE and transferred to nitrocellulose and autoradiographed (not shown). Phosphoaminoacid analysis was performed on the p66^{shc} polypeptide. Positions of the phosphoserine (S), phosphothreonine (T) and phosphotryosine (Y) markers are indicated.

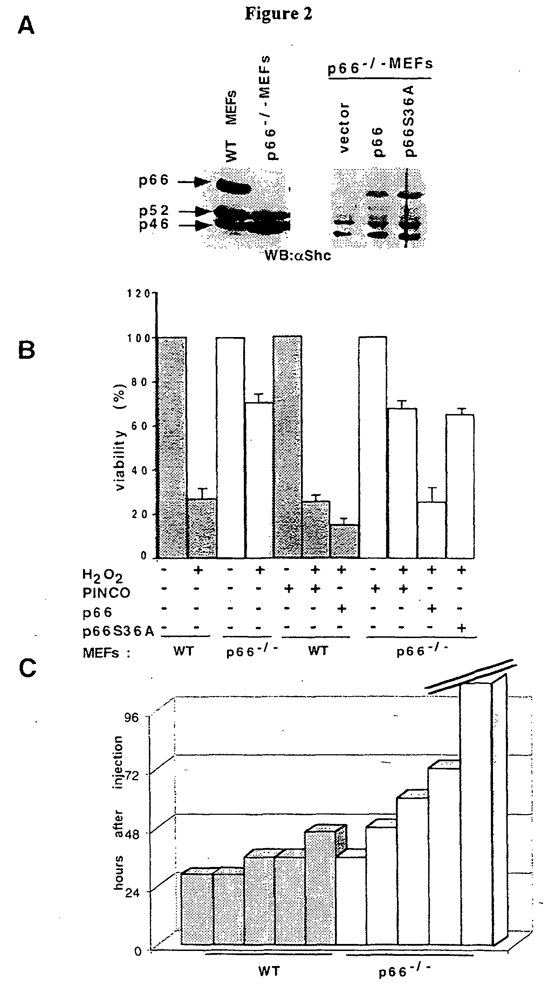

**Figure 2** shows p66^{shc} enhances stress oxidative response *in vitro* and *in vivo*. Figure 2A: Western blotting analysis of Shc expression in p66^{shc}+/+ or p66^{shc}-/- MEFs (left panel) and in p66^{shc}-/- MEFs transduced with vector alone, p66^{shc} or p66^{shc}S36A cDNAs (right panel). Figure 2B: Viability of MEFs after H₂O₂ treatment. Equal numbers (1.2 x 15⁵) of the indicated MEF cells grown in 100mm dishes in triplicate were infected with the PINCO retrovirus or with PINCO retroviruses expressing p66^{shc} or p66^{shc}S36A, as indicated, kept for additional 48 hrs to allow gene expression of exogenous cDNAs and exposed to 400mM H₂O₂ for 24 hrs. Cell viability was determined by trypan blue exclusion. Results are expressed as a percentage of viable cells with respect to H₂O₂ untreated controls and represent the mean of three independent experiments. Expression of p66^{shc} or p66^{shc}S36A did not significantly influence viability or growth rate of MEFs, as measured 48 hrs after viral infection (not shown).

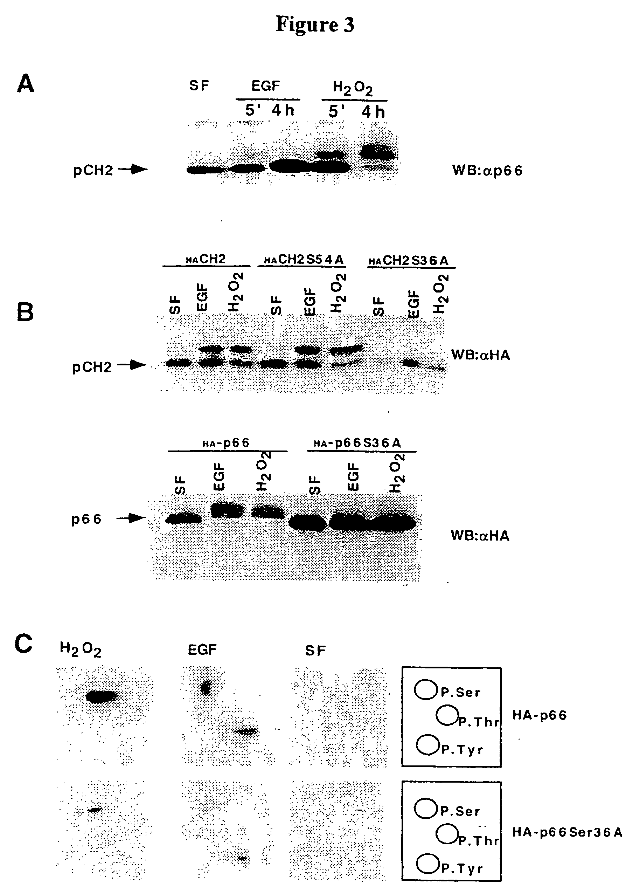

**Figure 3** shows mapping of p66^{shc} serine-phosphorylation sites. Figure 3A: Anti-CH2 western blots of lystates from MEFs transfected with vectors expressing the isolated CH2 region and then starved (SF) or treated with EGF or UV for 5 min or 4 hr, as indicated. The star indicates the shifted CH2 polypeptide. Figure 3B: The S36A or S54A mutations were introduced with the isolated CH2 region or the full-length p66^{shc}. The resulting cDNAs were HA-tagged, cloned with a pcDNA3 expression vector and transfected into MEFs. Cultures were treated as indicated and analysed by western blotting using anti-HA antibodies. Figure 3C: Phosphoaminoacid analysis of p66^{shc} and p66^{shc} S36A. MEFs were transfected with HA-p66^{shc} or HA p66^{shc} S36A expression vectors, kept in culture for 48 hrs labelled with 1mCi/ml[³²P] orthophosphate for 4 hr and treated with EGF or H₂O₂ for 5 min, as indicated, lysed and immunoprecipitated with anti-HA antibodies. Phosphoaminoacid analysis was performed on the HA-p66^{shc} or HA-p66^{shc}S36A polypeptides, as described in Fig. 1 legend.

**Figure 4** shows cumulative survival (Kaplan and Meier) or p66^{shc}+/+ (dashed line), p66^{shc}+/- (dotted line) and p66^{shc} -/- (solid line) mice. Survival of the p66^{shc}-/- mice was 71.4%.

**Figure 5** shows **(a)** the p66 cDNA nucleotide sequence (the ATG initiation site is underlined) and **(b)**, separated, the p66 amino acid sequence.

**Figure 6** shows a 13kb genomic region containing all the Shc coding exons which was characterised by restriction enzyme mapping and nucleotide sequence of all exon-intron boundaries and 5' regulatory regions.

**Figure 7** shows the construction of the targeting vector pBSp66ShcKO.

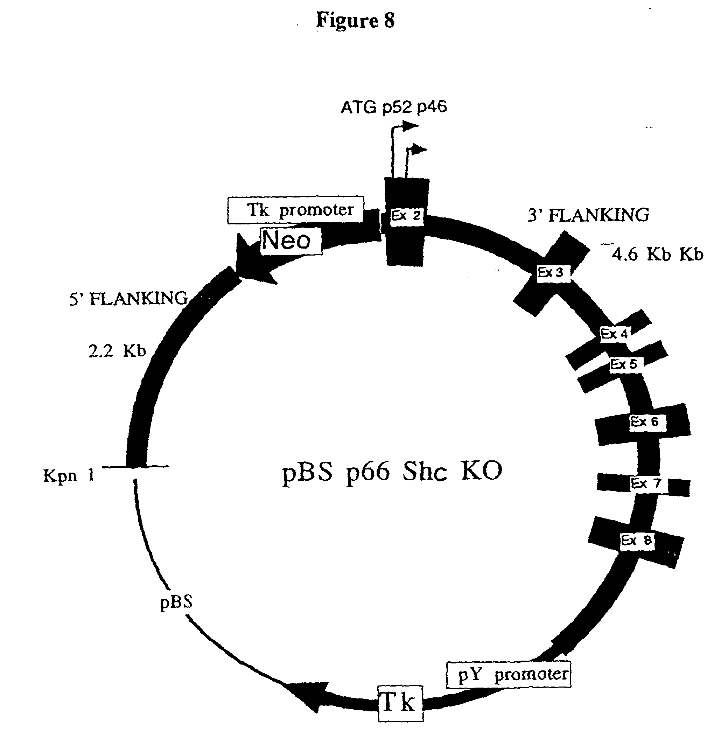

**Figure 8** shows the vector pBSp66ShcKO with a TK transcriptional unit cloned at its 3' end.

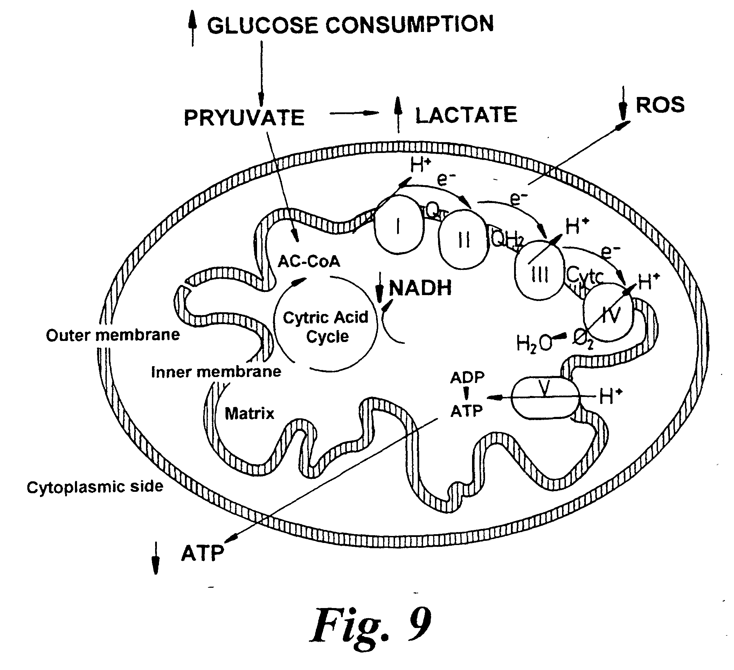

**Figure 9** shows multiple metabolic changes in p66^{Shc}-/- cells. In particular it shows the mechanism of p66 action and its metabolic effects, in particular the increased production of H₂0₂ and the consequent increase in concentration of Reactive Oxygen Species (ROS); and the increased production of ATP (at the cost of ROS). p66 is also confirmed to be located in the mitochondrial membrane and regulates a transition port and is involved in electron transfer.

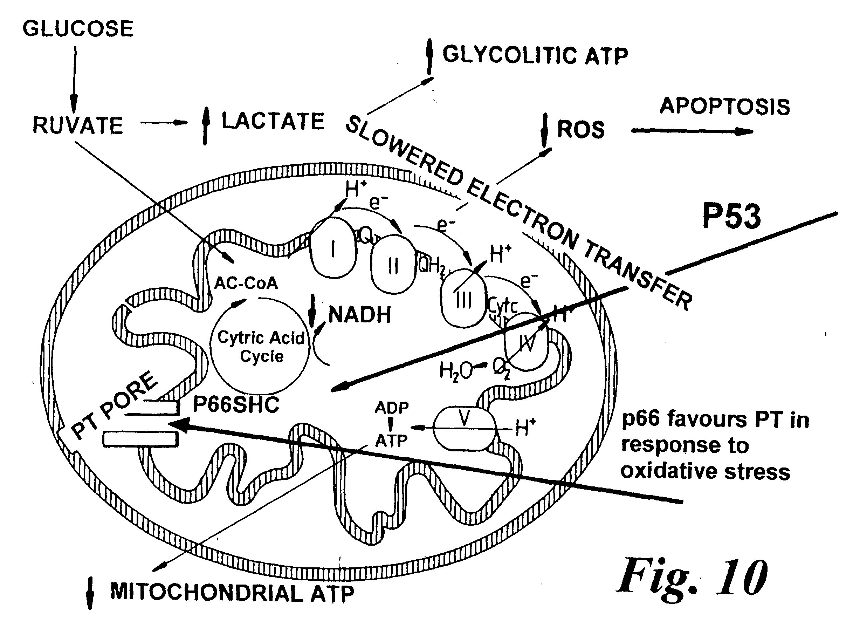

**Figure 10** shows regulation of mitochondrial activity by p66^{shc}.

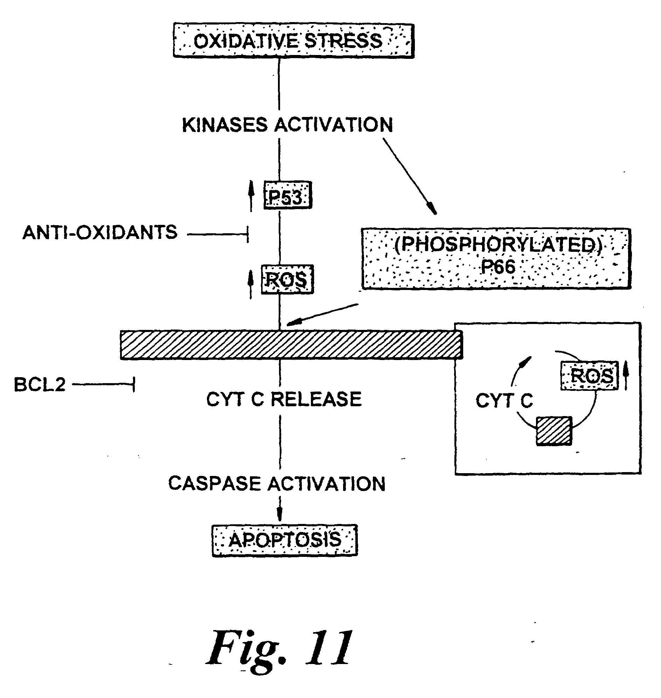

**Figure 11** shows the role of p66 in the p53-dependent apoptosis.

### Detailed Description

### 1) Cell lines, reagents and plasmid construction

Mouse embryo fibroblasts (MEFs) were isolated from 12 to 14 day embryos derived from p66^{shc}-/- mice and p66^{shc}+/+ mice and maintained in Earle's minimal essential medium supplemented with 10% fetal bovine serum. The S36A and S54A mutations were generated by standard PCR techniques. The p66^{shc}, p66^{shc}S36A, HA-CH2, HA-CH2S35A, HA-CH2S54A, HA-p66^{shc}, HA-p66^{shc}-S35A and HA-p66^{shc}S54A were cloned into the pCDNA3 or PINCO eukaryotic expression vectors (Claudio P.P. et al Cancer Res. 54, 5556-5560 (1994); Grignani, F. et al Cancer Res. 1, 14-19 (1998)). The antibodies used were: the anti-Shc polyclonal antibody which recognises the SH2 domain of all three Shc isoforms (Pelicci G. et al Cell 70, 93-104 (1992)); the anti p66 polyclonal antibody which recognises the p66^{shc} isoform (Migliaccio E. et al Embo J. 16, 706-716 (1997)); the anti-βactin polyclonal antibody, (Sigma Immuno Chemicals); the anti-HA monoclonal antibody; the anti-phosphotyrosine monoclonal antibody, (Santa Cruz Biotechnology).

### 2) Metabolic labeling immunoprecipitaion, Western blotting and phosphoaminoacid analysis.

For whole lysates, cells were directly lysed in SDS sample buffer (50mM Tris-HCL pH 6.8, 2% SDS v/v, 10% glycerol and 5% v/v β-mercaptoethanol) and boiled for 5 min. 50µg of total protein was analysed by SDS-PAGE. For immunoprecipitation, cells were lysed on ice in PY buffer (20mM Tris-HCL ph 7.8, 50mM NaCl, 30mM Na₄P₂O₇. 5mM sodium orthovanadate, 1% v/v Triton x-100 containing freshly added protease inhibitors: 1mM phenylmethyl sulfonhyl fluoride, 10µgml⁻¹ leupeptin and 5 mg ml⁻¹ aprotinin), appropriate antibodies were adsorbed on Protein A Sepharose (Pharmacia) and then incubated with cell lysates for 2hr at 4°C. Immunoprecipiates were recovered, resolved by 10% SDS-PAGE and transferred to nitrocellulose filters, as described elsewhere (Migliaccio E. et al Embo J. 16, 706-716 (1997)). Blots were blocked, probed with specific antibodies and immune complexes revealed by horseradish-peroxidase conjugated with specific secondary antiserum (Biorad) followed by enhanced chemiluminescence. For phosphoaminoacid analysis, cells were grown to confluence on 10 cm plates, starved in serum-free medium and labelled for 4h in 5 ml phosphate free DMEM containing 5% dialyzed FBS and 1mCi ml^{-1 32}P-orthophosphate. Cells were stimulated with 30ng ml⁻¹ EGF or 400µM H₂O₂, or irradiated with 50 J/m² UV, rinsed twice with ice cold PBS and lysed in PY buffer. p66^{shc} proteins were isolated by immunoprecipitation with anti-SHC or anti-HA antibodies and resolved by SDS-PAGE. p66^{shc} polypeptides were transferred to PVDF membranes and hydrolyzed in 6M HCl for 60 min at 110°C. The hydrolysis products were separated in the presence of phosphoserine, phosphothreonine and phosphotyrosine markers by SDS-PAGE at pH1.9 and pH3.5 in two dimensions on TLC plates.

### 3) Transfections, infections and cellular viability test.

MEFs were transfected with the LipofectaMINE PLUS Reagent GibcoBRL (average transfection efficiency 50%). For retroviral infections, the empty PINCO vector and recombinant PINCO vectors expressing p66^{shc} or p66^{shc}S36A cDNAs were transfected into the phoenix amphotropic packaging cell line and, after 48 hrs, supernatants were used to infect MEFs cells (1.2 x10⁵ cells/100mm dish). The efficiency of infection (GFP positive cells) was determined by FACS analysis 48 hr after infection. Viability was assessed by the trypan-blue dye exclusion test.

### 4) Statistical analysis

Survival functions were estimated by the Kaplan and Meier product limit method. Survival distributions were compared using the logrank test (Marubini E. et al New York, John Wiley & Sons (1995). All statistical calculations were performed using SAS/STAT Rel. 6.12 software (SAS Institute 1995).

### 5) Construction of p66^{shc} targeting vector and electroporation and selection of ES cells.

The targeting vector was constructed using standard cloning procedures. The plasmid was linearized with Kpn 1 before electroporation into ES cells. CJ7 ES cells were maintained on a monolayer of mitomycyn C inactivated, neomycin-resistant primary embryonic fibroblast. A suspension of 15 million trypsinized ES cells in PBS was electroporated with 25 µg of DNA of the linearized targeting vector by using the Bio-Rad gene pulser II apparatus with 240 V and 500 µF. Cells were plated immediately after transfection and allowed to recover for 24 hr before selection in medium 350 µg/ml Geneticin and 2 µM of ganciclovir. Cells were fed daily and after 9 days the resulting colonies were picked and cultivated singularly until extraction and freezing.

### 6) Southern blot analysis of ES cells and Mice.

To identify the mutated Shc allele, genomic DNA from ES cells and from the mouse tails was prepared by proteinase K Iysis and phenol-chloroform extraction, digested with Eco R1 and analyzed by Southern blot analysis. A 1.1.Kb Eco R1-Xba 1 fragment was used as external probe to discriminate between the WT 8 kb and the recombinant 3.5 kb allele bands (Fig 2).

### 7) Generation of mice carrying the disrupted p66 Shc allele.

Two different clones of targeted ES were used to generate chimeric mice. C57BL/6J blastocysts injected with 10-15 ES cells were transferred to pseudopregnant female mice. Chimeric mice, identifiable by agouti coat color, were mated with C57BL/6J mice. Offspring with agouti coat color were tested for the presence of the recombined allele by means of Southern blot analysis. Heterozygotes obtained from the crosses of the chimeras with 129Sv female mice were interbred to establish the colony of p66 -/- mice in the 129 genetic background. The mice were housed at a constant room temperature (22°C) and humidity (60%) with a 12 h light/dark cycle, with free access to standard mouse chow and tap water.

### Results

### 1) Construction of the p66shc targeting vector.

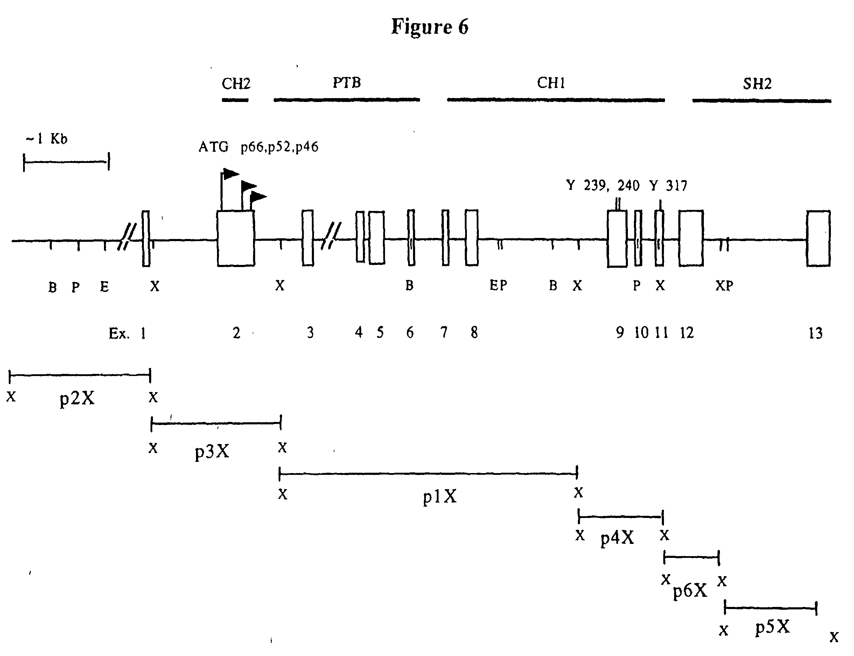

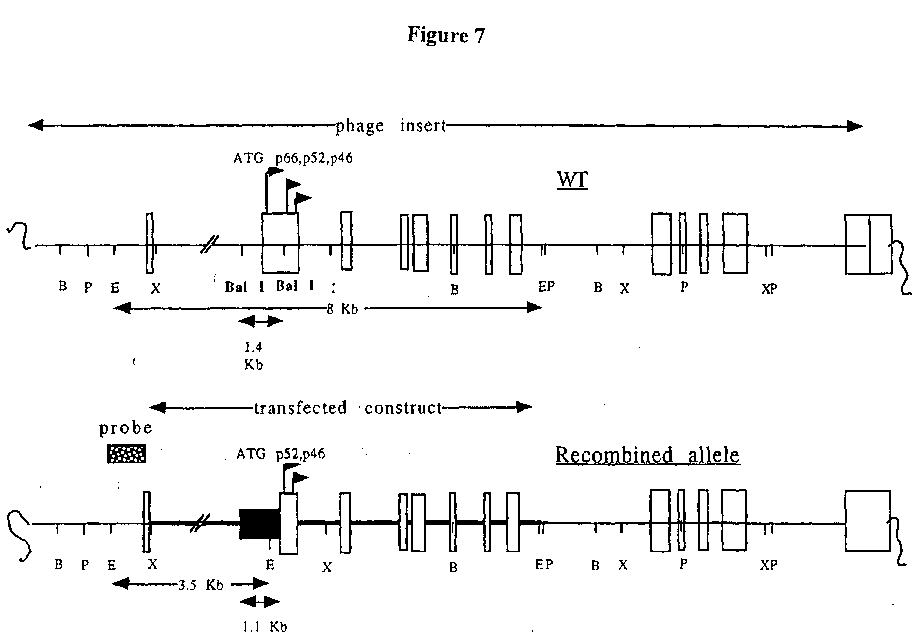

The present inventors have mutated the mouse Shc locus using conventional embryonic stem cell technology. The genomic Shc locus was isolated from a 129 mouse genomic library. A 13 Kb genomic region containing all the Shc coding exons was characterized by restriction enzyme mapping and nucleotide sequence of all exon-intron boundaries and 5' regulatory regions (Fig. 6). A positive/negative (G418/Ganciclovir) selection strategy was applied to introduce a mutation in the region of first coding exon that contains the p66 ATG. To construct the targeting vector (pBSp66ShcKO; Fig 7) we used the EcoR1 8 KB fragment containing exons 1-8 (Fig.7). The Bal 1 fragment containing the p66 specific start site (Fig.7) was substituted with the Neo transcriptional unit driven by the pY promoter. The resulting vectors contains 2,2 and 4,6 Kb flanking sequences at the 5' and 3' ends, respectively (Fig. 7). A TK transcriptional unit was cloned at its 3' end (Fig.3).

### 2) ES tranfection and selection.

CJ7 ES cells, provided by Dr, Vera Soares (Memorial Sloan Kettering Cancer Center, NY, USA) were transfected by electroporation, and resistant colonies were selected and screened for the deletion of the p66 first coding sequence by Southern blotting. The screening strategy was based on the EcoR1 site introduced in the targeted allele by the insertion of the Neo transcriptional unit (Fig.7) 24 ES clones of 150 analyzed showed one targeted Shc allele. Cytogenetic analysis of 9 ES clones revealed a normal modal number of chromosomes.

### 3) Generation of targeted mice.

Two ES clones were injected into C57BL/6J blastocysts, according to established procedures. Breeding of heterozygous (p66^{sch}+/-) yielded the expected frequency of homozygous animals. Analysis of the genotype of the animals was performed by Southern blotting of DNA extracted from the tails and confirmed by Western blotting of p66^{shc} expression in various tissues.

### 4) p66 is phosphorylated on Ser36 after H₂O₂ or UV stimulation.

Since Fos is transcritionally activated by a variety of environmental stresses (hydrogen peroxide: H₂O₂; ultraviolet irradiation: UV), the present inventors have analysed the modifications of p66 upon H₂O₂ or UV stimulation of mouse and human fibroblasts. Results revealed that p66 is markedly phosphoryated on serine upon H₂O₂/UV irradiation. Further, the present inventors mapped the major serine phosphoryation site to Ser 36. A Ser-Ala 36 p66 mutant is not phosphorylated by UV/H₂O₂ in vivo.

### 5) Erk1 and p38 mediates UV/H₂O₂ phosphorylation of p66

By using purified enzymes in *in vitro* kinase assays and either dominant negative kinases or specific inhibitors *in vivo,* the present inventors have demonstrated that p66 is phosphorylated by p38 and Erk1 upon UV or H₂O₂ stimulation.

Erk 1 (also known as MAPK), JNK and p38 are stress-induced kinases. To identify the kinase(s) responsible for the phosphorylation of p66 induced by oxidative stess in vivo, the present inventors analysed the extent of p66 phosphorylation after stress signals in MEFs expressing a JNK dominant negative kinase or in cells treated with various MAPK and p38 specific inhibitors [PD98059, which prevents activation of Erk 1 by Raf; SB203580 which specifically inhibits the p38 Map kinases]. Results showed that SB203580 and PD98059, but not the JNK dominant negative kinase, prevented p66 phosphorylation by oxidative stress signals (H₂O₂), indicating that p66 is phosphorylated in vivo by Erk1 and p38, but not by JNK.

The present inventors then reconstructed the p66 phosphorylation in vitro by using recombinant Erk1 or JNK and the bacterially expressed p66 CH2 region (CH2 was expressed in bacteria as GST-fusion protein). Erk1, but not JNK, was unable to in vitro phosphorylate the p66 CH2 region. Phosphorylation was specific, as shown by the finding that, in the same assay, Erk1 was unable to phosphorylate the p66 CH2 region when the S36A mutation was introduced.

### 6) p66 modulates the oxidative stress response in vitro

H₂O₂ treatment induces fibroblast cell death. The present inventors have demonstrated that: i) overexpression of p66 in wild-type. MEFs increases cell death induced by H₂O₂; ii) p66-/- MEFs are more resistant to H₂O₂-induced cell death than wild-type controls *in vivo*. Paraquat is a pesticide that kills mice by inducing oxidative damage. The present inventors have further demonstrated that p66-/- mice are more resistant to paraquat treatment than littermates.

### 7) p66 regulates the p16, p53 and p21 response

Since environmental stresses activates the p16 - p53-p21 signalling pathways, the present inventors have further investigated whether p66 interferes with p16-p53-p21 activation by H₂O₂. Results revealed that p16, p53 and p21 activation are lost in p66-/- cell upon H₂O₂ treatment.

### 8) p66 is a tumour supressor

*In vitro,* the stimulatory effect of p66 on the p53-p21 pathway suggests that it might play a role in the cellular response on oncogenic stimuli. Therefore, the present inventors have evaluated the effects of p66 on the response of primary fibroblasts on the oncogenic RASV12 mutant. RASV12 induces senescence of wild-type MEFs, as a consequence of p53-p21 activation. Expression of RASV12 into p66-/- MEFs induced cellular transformation. Furthermore, the present inventors have demonstrated that p53 and p16 are unable to induce senescence of mouse p66-/- fibroblasts.

### 9) p66 mediates aging

The results presented herein demonstrate that p66 is involved in the cellular response to stresses (environmental and oncogenic). The present inventors therefore considered whether p66 is also involved in mediating aging. To do this, they evaluated the survival of p66-/- mice. Of the many mice that were born in August 1996, 14+/+, 8+/- and 15-/- were not sacrificed and kept for survival analysis. Evaluation after 28 months (December 1998) revealed

| | |
|---|---|
| +/+ | 0/14 survivors 0% |
| +/- | 3/8 survivors 37% |
| -/- | 11/15 survivors 73% |

### 10) P66-/- MEFs are resistant to p53-induced apoptosis

To analyse the molecular mechanisms underlying the increased resistance of p66-/- MEFs to oxidative stress-induced apoptosis, the present inventors analysed the function of p53 in p66-/- MEFs (versus wt MEFs) following H₂O₂ treatment.

P53 is a tumour suppressor gene that functions as a transcription factor. P53 is also a critical component of cellular mechanisms that respond to a variety of different environmental stresses, including DNA damage, hypoxia or oxidative stress, and which lead to cell cycle arrest or apoptosis. Stress-induced p53 activation involves increased protein stability and post-translational modifications including phosphorylation and acetylation.

H₂O₂ treatment induced up-regulation of p53 into wt as well as p66-/- MEFs, implying that the mechanisms that underlie increased p53 stability upon oxidative stress are not affected by p66 expression. However, overexpression of p53 (using retroviruses or adenoviruses) into p66-/- MEFs was unable to induce apoptosis, indicating that the p53-downstream pathway is altered in the absence of p66.

To understand the mechanisms underlying the loss of p53 activity into p66-/- cells, the inventors measured the potential of p53 to act as a transcription factor in p66-/- cells. To this end, they performed transactivation experiments using p53 and a p53-target promoter into p66-/- and wt MEFs. Results showed that the transcriptional activity of p53 is retained into p66-/- MEFs. This finding suggest that the inability of p53 to execute the apoptotic process into p66-/- MEFs might be due to alterations of the activity of (some) p53-target genes.

Little is known on the p53-target genes which are involved in the execution of the many p53 biological activities (apoptosis, senescence, cell cycle arrest). In the case of apoptosis, it has been recently demonstrated that p53 activate a set of genes (so called PIGs; p53 inducible genes) that are involved in the control of ROS metabolism. Indeed, p53 activation results in the transcriptional activation of these genes, increased intracellular concentrations of ROS, caspase activation and apoptosis. -

To test whether this intracellular pathway is altered into p66-/- MEFs, the inventors have measured the intracellular concentration of ROS into p66-/- and wt MEFs following p53 overexpression (using fluorescent dyes, such as the DCFDA). Results showed that the p53-induced peak of intracellular ROS following p53 expression is lost into the p66-/- MEFs.

Together, these results indicate that p66 regulates ROS metabolism and is a crucial effector of p53-mediated apoptosis.

### 11) P66-/- MEFs are resistant to Ras-induced replicative senescence

p53 regulates a checkpoint response to oncogenic signals: expression of oncogenic mutants of Ras in primary cells activate p53, resulting in premature senescence, whilst the absence of functional p53 is sufficient for oncogenic transformation of murine fibroblasts. P53, indeed, is mutated at high frequency in many tumour types, implying that its action is central to tumour development. The mechanisms through which cells sense hyperproliferation and which lead to p53 activation and cellular senescence remain, however, poorly understood. It has been recently demonstrated that Ras-induced senescence involved generation of ROS, implying that ROS are also involved in the execution of the senescence program by p53.

Therefore, The inventors have analysed whether Ras is able to induce cellular senescence into p66-/- MEFs. Results showed that p66-/- MEFs are resistant to Ras-induced senescence, suggesting that the intracellular mechanisms that regulate the response to oncogenic signals is altered into p66-/- cells.

To investigate whether p66-/- MEFs are more susceptible to tumour formation, the present inventors analysed the ability of Ras-expressing p66-/- MEFs to form colonies into methylcellulose (a property of transformed cells), as compared to p53-/- MEFs. While Ras expression induced transformation of p53-/- MEFs, it was unable to do so into p66-/- MEFs. Likewise, p66-/- mice did not show increased frequency of spontaneous or UV (or TPA)-induced tumours.

The inventors conclude that p66 expression is indispensable for the capacity of p53 to induce apoptosis (following oxidative stress) or senescence (following Ras expression). However, the loss of p66 does not mimic the loss of p53 with respect to the tumour-prone phenotype, suggesting that other pathways are activated by p53 following oncogenic signals.

### Potential applications.

P66 is a downstream target of p53 in the execution of the p53-mediated apoptosis and cellular senescence. Manipulations of p66, therefore, can be used to restore the p53 function into p53 null cells. A potential application of this finding is the possibility to activate the apoptotic or senescence pathways in tumour with non-functional p53 (the p19-p53 pathways are non-functional in the vast majority of tumours). Agonist of p66 can be envisioned as putative tumour suppressors. Any molecule that would prevent p66 de-phosphorylation or kinases that induce p66 phosphorylation are potential device for tumour treatment.

### 12) The intracellular concentration of ROS (reactive oxygen species) in p66-/- MEFs is decreased.

The above-mentioned results suggest that p66 is involved in the regulation of ROS metabolism. Therefore the inventors have measured the intracellular concentrations of ROS in cultures of wt and p66-/- MEFs (using fluorescent dyes, such as the DCFDA). Results showed that the intracellular concentration of ROS is consistently decreased in p66-/- MEFs (of about one third).

### Potential implications.

Oxidative stress has been implicated in the generation of many human diseases. ROS induce damage to a variety of macromolecules, such as proteins (disulphide bond formation); lipids (peroxidation); DNA (mutations). These alterations are thought to mediate the phenotypic traits of aging and to be implicated in the pathogenesis of different diseases (atherosclerosis; ischemic heart disease; Parkinson, Alzheimer, vascular complications of diabetes). Inhibitors of the p66 function might be used in the treatment of these diseases.

### 13) P66 is a mitochondrial protein .

The present inventors have investigated the mechanisms underlying the regulation of ROS production by p66. ROS are mainly produced by mitochondria, as a consequence of the minimal, yet physiological, uncoupling of the electron transfer during oxidative phosphorylation. Therefore, the inventors have first evaluated whether p66 is a mitochondrial protein. Elector microscopic, immunofluorescence and cell fractionation studies (using anti-p66 specific polyclonal and monoclonal antibodies) revealed that p66 is a mitochondrial protein, located in the inner membrane of the mitochondria.

### 14) The decrease of the mitochondrial Δψ (mΔψ) following H2O2 treatment is slowered in p66-/- MEFs .

To assess if the different survival response to H2O2 treatment of p66-/- MEFs also reflects a different mitochondrial response, the inventors tested the functionality of the mitochondria in the p66-/- and WT MEFs after treatment with H₂O₂.

The mitochondrial electric potential (mΔψ) is a direct measure of the electro-chemical gradient developed by the activity of the electron transfer chain complexes and the resulting proton pumping. mΔψ was, therefore, measured as indicator of mitochondrial activity after H₂O₂ treatment, using tetramethylrhodamine methyl ester (TMRM). TMRM is a lipophilic cationic fluorescent probe which is accumulated in the mitochondria according to the Nerst's equation. The higher is the mΔψ, the more intensive is the signal resulting from the fluorescence of the TMRM, thereby allowing determination of mΔψ variations in cell culture systems.

The inventors found that the reduction of the mΔψ after 10 minutes from the addition of 400 µM of H₂O₂ is about 50% in the wt MEFs and only 20% in the p66-/- MEFs. This result indicates that mitochondria in p66-/- are less damaged by H₂O₂ with respect to those of wt cells.

### 15) Isolated mitochondria from p66-/- mouse livers are more coupled following stimulation with different substrates

In order to determine if mitochondrial respiratory performances are altered by the absence of the p66 protein, purified mitochondria from WT and p66-/- mouse livers were analysed for their respiratory activity. *In vitro* quantitation of oxigen consumption by the mitochondrial suspensions was performed using an oxigen sensitive electrode. The inventors measured both basal and stimulated respiration following administration of ADP, succinate, aspartate, malate, dinitrophenol, and calculated the state 3 / state 4 ratio of WT and p66-/mitochondria. The inventors consistently found higher values of this ratio for the p66-/- as compared to the wt mitochondria. These findings suggest that mitochondria extracted from p66-/- livers are in a better "coupled state".

### 16) Swelling as a measure of Permeability Transition induced by H₂O₂ is lowered in isolated p66-/- respect to the WT mouse liver mitochondria .

Mammalian mitochondria possess an inner membrane channel, the permeability transition pore (MTP). Opening of the MTP is responsible for the permeability transition (PT), a sudden increase in permeability to solutes with molecular masses approximately 1500 Da (experimentally obtained after Ca²⁺ accumulation in the presence of a variety of so-called "induging agents"). The PT is the primary event in apoptosis, and, together with mitochondrial depolarization, it represents the "central executioner" in the apoptotic cascade. PT results in a modification of the mitochondrial volume and in a consequent swelling of the organelle.

It is possible to analyse the relative MTP opening probability and the resulting PT by measuring the variation of absorbance of the swollen using a spectrophotometer at 540 nm wavelength. The inventors analysed the "swelling" response of isolated liver mitochondria from WT and p66-/- mice after different PT stimuli. An impairment/delay of the swelling was detected in the p66-/- mitochondria after treatment with 100 µM H₂O₂. This finding indicates that the MTP in the p66-/- mitochondria-is less sensitive to oxidation and its consequent opening induced by H₂O₂ suggesting that the regulation of the opening of the MTP is one of the events controlled by p66 in the mitochondria. p66, therefore, would function favouring the opening of the pore, thereby determining a more fragile and uncoupled mitochondria. This would result into less efficient respiratory performance, more active production of ROS and better apoptotic response to oxidative stress.

### Potential implications

Since p66 controls the function of the permeability pore in mitochondria and, indirectly, the efficiency of electron transfer and ROS production, alterations of the p66 function are expected to allow manipulation of metabolism and apoptosis.

### 17) Old Lung cellular density is higher in the p66-/-

All levels of organisation of an organism, from molecules to complete organ systems, undergo senescence and a large body of evidence indicates that free radicals are the primary cause of this phenomenon. The resulting oxidative stress induced aging is a process strictly associated with the decrease of functional capacities in all parts of the body and even if it does not clearly define the causes of death occurring at the end of the life span of an organism, it is generally charged to this degenerative senile syndrome.

Some studies suggest that kidney and heart failure are the most common cause of death at very old age in humans as in other mammals but also disfunctions in other organs due to aging significantly contribute to the death at the end.

In particular, in the lung, the volume/weight ration of inflated dried lung parenchyma increase significantly with aging resulting in the so-called "aging-lung emphysema" or "senile emphysema". The oxidative stress accumulated during aging induced programmed cell death which may contribute to the thinning of the alveolar septa observed with aging. The examination of aged (more then 1 year old) p66-/- and wild type mouse lungs reveals that p66-/- lung weights and sizes are larger than wild type. Histological analysis of these lungs shows a general pattern of higher cellular density and in particular thicker alveolar walls are present in the p66-/- lung.

The present inventors, therefore have concluded that the delayed thinning of the alveolar walls and the consequent early appearance of signs of senile-emphysema in wild type mice could suggest a possible mechanistic explanation which may be extended to other organs, for the prolonged life observed in the absence of p66.

### Potential implications

Regardless of the contribution of delayed senile emphysema to the increased lifespan of p66-/- mice, these findings indicate that the lung is a target organ for p66 functions. Inhibition of p66 function into the lung, may therefore lead to increased functionality of the lung and provide new treatments for emphysema.

### Discussion

To investigate its role in the cellular stress response, the present inventors analysed the extent of p66^{shc} tyrosine-phosphorylation in mouse embryo fibroblasts (MEFs) treated with UV or H₂0₂, as compared to the effects of treatment with growth factors, such as the epidermal-growth factor (EGF). Anti-p66 immunoprecipitates from lysates of untreated and EGF-, UV-, or H₂O₂-stimulated fibroblasts were immunoblotted with anti-phosphotyrosine antibodies. EGF stimulation induced a marked increase in the phosphotyrosine content of p66^{shc}, which was maximal after 5 min. Neither UV nor-H₂O₂ treatment induced significant tyrosinephosphorylation of p66^{shc} (Fig.1B). However, Western blotting of the same lysates with anti-Shc antibodies revealed a marked gel retardation of the p66^{shc} polypeptides after 5 min and 4 hours treatment with UV or H₂O₂, consistent with other post-translational modifications of p66^{shc} induced by these agents (Fig.1C). Therefore, p66^{shc} phosphorylation was analyzed by phosphoaminoacid analysis (Fig.2C). p66^{shc} polypeptides from serum-starved cells were phosphorylated primarily on serine. UV (Fig.2C) and H₂O₂ (not shown) induced a marked increase in the level of phosphoserine and had no effect on phosphotyrosine. In contrast, EGF induced a marked increase in the level of phosphotyrosine and a modest increase in phosphoserine (Fig.2C). It appears, therefore, that p66^{shc} is involved in the intracellular transduction pathways of both environmental stresses and growth factors, albeit with distinct functions, since UV and H₂O₂ induced rapid and persistent serine-phosphorylation, while EGF induced rapid and transient tyrosine-phosphorylation.

To investigate the functional role of p66^{shc} in the stress oxidative response, the present inventors next analysed the effects of p66^{shc} overexpression or p66^{shc} ablation on the cellular response of MEFs to H₂O₂. MEFs were derived from mice carrying a targeted mutation of the Shc locus that disrupted the exon encoding the p66 CH2 region, without affecting the p52^{shc}/p46^{shc} coding sequences (M. Giogio et al : submitted for publication). p66^{shc}+/+ MEFs from wild-type mice with otherwise identical genetic background were used for comparison. As expected, expression of p66^{shc} was normal in p66^{shc}+/+ MEFs while was undetectable in p66^{shc}-/- MEFs; expression p52^{shc}/p46^{shc} was identical in p66^{shc}+/+ and p66^{shc}-/- MEFs. p66^{shc}+/+ MEFs were susceptible to H₂0₂ treatment, with more than 70% of cells being killed after 24 hours exposure to 400 µM H₂0₂. Overexpression of p66^{shc} rendered p66^{shc}+/+ more susceptible to H₂0₂ treatment (approximately 85-90% cell death after 24 hours). In contrast, p66^{shc}-/- MEF cells were more resistant to killing by the same dose of H₂0₂ and more than 70% of these cells survived after 24 hours of H₂0₂ treatment (Fig.2B). Expression of the p66^{shc} cDNA into p66^{shc}-/- cells restored a normal response to H₂0₂ (Fig.2B).

The present inventors then examined the ability of p66^{shc}-/- mice to resist oxidative stress *in vivo.* To this end, mice were treated with paraquat, which, upon intake by the cell, generates superoxide anion. At a dosage of 70mg/kg, 5 of 5 p66^{shc}+/+ mice died within 48 hours after paraquat administration. In contrast, out of 5 p66^{shc}-/- treated mice, two died within the first 48 hours, two after approximately 72 hours and 1 survived for several weeks (Fig.2C). Together, these results point to a function of p66^{shc} in the cellular stress oxidative response.

To investigate whether p66^{shc} participates in the cellular stress response as a cytoplasmic transducer of stress signals, The present inventors analysed the potential of a serine-phosphorylation defective mutant of p66^{shc} to rescue the impaired stress oxidative response of p66^{shc}-/- MEFs. The p66^{shc} CH2 region-probably contains the p66^{shc} major serine-phosphorylation site(s), as suggested by the gel-mobility shift induced by H₂O₂ and EGF, when the CH2 was expressed in cultured cells as isolated domain (Fig.3A). The CH2 region contains three serine residues with a consensus sequence for serine/threonine kinase phosphorylation (S28, S36 and S54) (Davis, J. J. Biol. Chem. 268, 14553-14556 (1993)). Alanine substitution of S36 abrogated the gel-mobility shift of both isolated CH2 and full-length p66^{shc} induced by H₂O₂ (Fig.3B). Phosphoaminoacid analysis of the p66^{shc} and p66^{shc}S36A polypeptides revealed a marked increase in the level of phosphoserine induced by H₂O₂ in the p66^{shc}, but not in the p66^{shc}S36A mutant (Fig.3C), thereby confirming that S36 is the p66^{shc} major serine phosphorylation site.

The present inventors then expressed the p66^{shc}S36A mutant into p66^{shc}-/- MEFs and evaluated its effect on the stress oxidative response. As shown in Fig. 2B, p66^{shc}S36A was unable to restore a normal response to H₂O₂. Instead, it conferred further resistance to H₂O₂-induced cell death, probably through a dominant negative effect on the stress response-signalling pathway. Together, these results indicate that p66^{shc} acts as a signal transducer in the cellular response to oxidative stress.

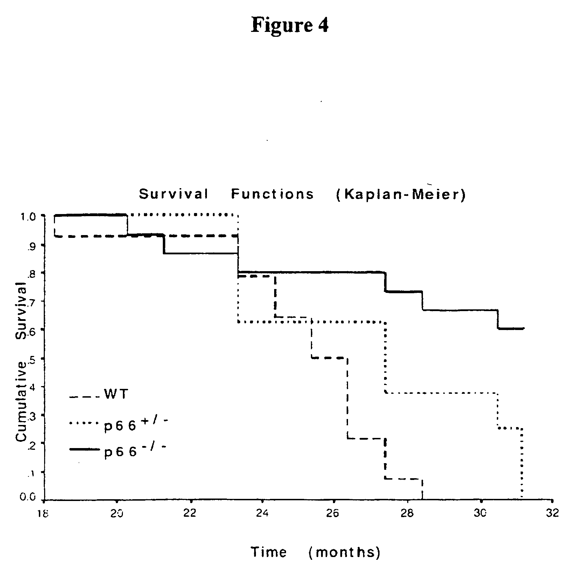

Enhanced resistance to environmental stress correlates with prolonged lifespan in invertebrates. In S.Cerevisiae, deletions of RAS1 (Sun, J. et al J. Biol. Chem. 269, 18638-18645 (1994); Kale, S. P. et al Dev. Genet. 18, 154-160 (1996)) or mutations in the SIR4 (Kennedy, B. K. et al Cell 80, 485-496 (1995)) locus increase lifespan and resistance to starvation, ethanol and heat shock or UV, respectively. In C. *elegans*, mutants of genes of the dauer signalling pathway, such as age-1 and daf-2, survive longer and are more resistant to oxygen radicals, heat and UV (Murakami, S. et al Genetics, 143, 1207-1218 (1985); Larsen, P. L. et al Genetics 139, 1576-1583 (1995)). In *D.melanogaster*, selection for late-life fitness is associated with greater resistance to environmental stresses (Service, P. M. et al Physiol, Zool. 58, 380-389 (1985); Service P.M. Physiol Zool. 60, 321-326 (1987); Arking R. et al Dev. Genet. 12 362-370 (1991)), and hypomorphic mutants of the mth locus live 35% longer and are more resistant to dietary paraquat and starvation (Lin, Y. J. et al Science 282, (1998). The present inventors, therefore, retrospectively analysed the effects of the p66^{shc} mutation on lifespan. 37 mice born on August 1996 from p66^{shc}+/-heterozygous parents were not sacrificed and maintained under identical conditions of stability. They consisted of 14 p66^{shc}+/+, 8 p66^{shc}+/- and 15 p66^{shc}-/- mice. After 28 months of observation, all the wild-type animals had died (median survival of 25.37±0.63 months), while 3 of the 8 heterozygous (37%) and 11 of the 15 homozygous (73%) were still alive. The remaining 3 p66^{shc}+/- died after additional two months (median survival of 27.40±2.819). 3 p66^{shc}-/- mice also died after two months; the remaining 9 are still alive (lifespan more than 31 months). The comparison of survival curves obtained by the Kaplan and Meier method (Marubinii, E. et al Valsecchi, M.G. New York, John Wiley & Sons (1995))(Fig.4) showed a highly significant difference between the three groups (log-rank p=0.0002). Cumulative survival did not differ significantly between wild type and heterozygous (p=ns [0.057]). The cumulative survival in the p66^{shc}+/+ group was 71.4% (p<0.01 vs p66^{shc}+/- and p66^{shc}+/+). Therefore, it appears that homozygous mutation of p66^{shc} correlates with prolonged survival in mice.

The results presented herein are consistent with a model in which lifespan is determined as a result of the increased ability to resist or repair environmental damage. p66^{shc} is part of a signal transduction pathway, which is activated by environmental stresses (H₂O₂ or UV) and whose mutation increases stress resistance and lifespan. Biochemical and genetic investigation of the p66^{shc} signalling pathway should lead to better understanding of mechanisms relevant to aging in mammals.

## Claims

1. A nucleic acid molecule comprising a p66^{shc} coding sequence, the coding sequence consisting of the coding sequence of Figure 5 incorporating a mutation such that, in the protein encoded by the coding sequence, residue S36 is absent or replaced by a different amino acid residue.

2. A nucleic acid molecule according to claim 1 wherein S36 is replaced by alanine (p66^{shc}S36A).

3. A polypeptide encoded by a nucleic acid molecule according to claim 1 or claim 2.

4. A replicable vector comprising nucleic acid according to claim 1 or claim 2 operably linked to control sequences to direct its expression.

5. A host cell transformed with a vector according to claim 4.

6. A method of producing a modified p66^{shc} polypeptide comprising culturing a host cell according to claim 5 so that the p66^{shc} polypeptide is produced.

7. A method of producing a modified nucleic acid molecule comprising a p66^{shc} coding sequence, the method comprising modifying the coding sequence of Figure 5 such that, in the protein encoded by the coding sequence, residue S36 of the protein encoded by the sequence of Figure 5 is absent or replaced by a different amino acid residue.

8. A method of producing a modified p66^{shc} polypeptide, comprising modifying a nucleic acid sequence as described in claim 7, and expressing the modified nucleic acid in a host cell.

9. An in vitro method of disrupting the p66shc signal transduction pathway in a cell comprising contacting said cell with a nucleic acid molecule capable of hybridizing to nucleic acid encoding p66^{shc} thereby preventing said p66^{shc} expression.

10. An in vitro method of increasing resistance in cells to oxidative stress comprising the step of disrupting the p66^{shc} signalling pathway.

11. An in vitro method according to claim 10 wherein said step of disrupting the p66^{shc} affects the susceptibility of p66^{shc} to phosphorylation.

12. An in vitro method according to claim 10 or claim 11 wherein said step of disrupting the p66^{shc} pathway causes a mutant p66^{shc} polypeptide to be expressed such that the serine residue S36 of the protein encoded by the sequence of Figure 5 is absent or replaced by a different amino acid residue.

13. An in vitro method according to claim 12 wherein the residue corresponding to S36 is replaced by alanine.

14. An in vitro method of increasing resistance in cells to oxidative stress comprising the step of disrupting the p66^{shc} signalling pathway by contacting the cell with an antibody capable of specifically binding to the p66^{shc} polypeptide such that subsequent binding of p66^{shc} to other proteins is prevented.

15. An in vitro method of increasing resistance in cells to oxidative stress comprising the step of disrupting the p66^{shc} signalling pathway by contacting said cell with an antisense oligonucleotide capable of hybridising to the nucleic acid encoding the p66^{shc} polypeptide.

16. Use of an antisense oligonucleotide capable of specifically hybridising to p66^{shc} nucleic acid in the preparation of a medicament for increasing resistance in cells to oxidative stress.

17. Use according to claim 16 wherein the p66^{shc} nucleic acid sequence is shown in Fig. 5.

18. Use of an antibody capable of specifically binding to a p66^{shc} polypeptide or fragment thereof in the preparation of a medicament for increasing resistance in cells to oxidative stress.

19. Use of a vector comprising nucleic acid encoding p66^{shc} polypeptide in the preparation of a medicament for increasing resistance to tumour formation in a tissue, said vector being capable of incorporating said nucleic acid into the genome of the cells of the tissue.

## Patentansprüche

1. Nucleinsäuremolekül, umfassend eine p66^{shc}-Kodiersequenz, wobei die Kodiersequenz aus der Kodiersequenz aus Fig. 5 besteht, die eine Mutation umfasst, sodass in dem durch die Kodiersequenz kodierten Protein der Rest S36 nicht vorhanden oder durch einen anderen Aminosäurerest ersetzt ist.

2. Nucleinsäuremolekül nach Anspruch 1, worin S36 durch Alanin ersetzt ist (p66^{shc}S36A).

3. Polypeptid, für das ein Nucleinsäuremolekül nach Anspruch 1 oder Anspruch 2 kodiert.

4. Replizierbarer Vektor, der Nucleinsäure nach Anspruch 1 oder Anspruch 2, operabel an Kontrollsequenzen zur Steuerung seiner Expression gebunden, umfasst.

5. Wirtszelle, die mit einem Vektor nach Anspruch 4 transformiert ist.

6. Verfahren zur Herstellung eines modifizierten p66^{shc}-Polypeptids, umfassend das Kultivieren einer Wirtszelle nach Anspruch 5, sodass die p66^{shc}-Kodiersequenz produziert wird.

7. Verfahren zur Herstellung eines modifizierten Nucleinsäuremoleküls, das eine p66^{shc}-Kodiersequenz umfasst, wobei das Verfahren das Modifizieren der Kodiersequenz aus Fig. 5 umfasst, sodass in dem durch die Kodiersequenz kodierten Protein der Rest S36 des durch die Sequenz aus Fig. 5 kodierten Proteins nicht vorhanden oder durch einen anderen Aminosäurerest ersetzt ist.

8. Verfahren zur Herstellung eines modifizierten p66^{shc}-Polypeptids, umfassend das Modifizieren einer Nucleinsäuresequenz wie in Anspruch 7 beschrieben und das Exprimieren der modifizierten Nucleinsäure in einer Wirtszelle.

9. In-vitro-Verfahren zum Unterbrechen des p66^{shc}-Signaltransduktionsweges in einer Zelle, umfassend das Kontaktieren der Zelle mit einem Nucleinsäuremolekül, das in der Lage ist, an für p66^{shc} kodierende Nucleinsäure zu hybridisieren, wodurch die p66^{shc}-Expression verhindert wird.

10. In-vitro-Verfahren zur Steigerung der Resistenz in Zellen gegen oxidative Belastung, umfassend den Schritt des Unterbrechens des p66^{shc}-Signalisierungsweges.

11. In-vitro-Verfahren nach Anspruch 10, worin der Schritt des Unterbrechens des p66^{shc} die Empfindlichkeit von p66^{shc} gegenüber Phosphorylierung beeinflusst.

12. In-vitro-Verfahren nach Anspruch 10 oder Anspruch 11, worin der Schritt des Unterbrechens des p66^{shc}-Weges die Expression eines derartig mutierten p66^{shc}-Polypeptids verursacht, dass der Serinrest S36 des durch die Sequenz aus Fig. 5 kodierten Proteins nicht vorhanden oder durch einen anderen Aminosäurerest ersetzt ist.

13. In-vitro-Verfahren nach Anspruch 12, worin der S36 entsprechende Rest durch Alanin ersetzt ist.

14. In-vitro-Verfahren zur Steigerung der Resistenz von Zellen gegen oxidative Belastung, umfassend den Schritt des Unterbrechens des p66^{shc}-Signatisierungsweges durch Kontaktieren der Zelle mit einem Antikörper, der in der Lage ist, sich spezifisch an das p66^{shc}-Polypeptid zu binden, sodass eine spätere Bindung von p66^{shc} an andere Proteine verhindert wird.

15. In-vitro-Verfahren zur Steigerung der Resistenz von Zellen gegen oxidative Belastung, umfassend den Schritt des Unterbrechens des p66^{shc}-Signalisierungsweges durch Kontaktieren der Zelle mit einem Antisense-Oligonucleotid, das in der Lage ist, an die für das p66^{shc}-Polypeptid kodierende Nucleinsäure zu hybridisieren.

16. Verwendung eines Antisense-Oligonucleotids, das in der Lage ist, spezifisch an p66^{shc}-Nucleinsäure zu hybridisieren, zur Herstellung eines Medikaments zur Steigerung der Resistenz von Zellen gegen oxidative Belastung.

17. Verwendung nach Anspruch 16, worin die p66^{shc}-Nucleinsäuresequenz in Fig. 5 dargestellt ist.

18. Verwendung eines Antikörpers, der in der Lage ist, sich spezifisch an ein p66^{shc}-Polypeptid zu binden, oder eines Fragments davon zur Herstellung eines Medikaments zur Steigerung der Resistenz von Zellen gegen oxidative Belastung.

19. Verwendung eines Vektors, der für p66^{shc}-Polypeptid kodierende Nucleinsäure umfasst, zur Herstellung eines Medikaments zur Steigerung der Resistenz gegen Tumorbildung in einem Gewebe, wobei der Vektor in der Lage ist, die obige Nucleinsäure in das Genom der Zellen des Gewebes einzubinden.

## Revendications

1. Une molécule d'acide nucléique comprenant une séquence de codage de p66 ^{shc}, la séquence de codage se composant de la séquence de codage de la Figure 5 incorporant une mutation telle que, dans la protéine codée par la séquence de codage, le résidu S36 est absent ou est remplacé par un résidu d'acide aminé différent.

2. Une molécule d'acide nucléique selon la revendication 1 dans laquelle S36 est remplacé par de l'alanine (p66^{shc}S36A).

3. Un polypeptide codé par une molécule d'acide nucléique selon la revendication 1 ou la revendication 2.

4. Un vecteur réplicable comprenant de l'acide nucléique selon la revendication 1 ou la revendication 2 relié de manière opérable à des séquences de commande pour diriger son expression.

5. Une cellule hôte transformée par un vecteur selon la revendication 4.

6. Un procédé de production d'un polypeptide de p66^{Shc} modifié comprenant la mise en culture d'une cellule hôte selon la revendication 5 de manière que soit produit le polypeptide de p66^{Shc}.

7. Un procédé de production d'une molécule d'acide nucléique modifié comprenant une séquence de codage de p66^{shc}, le procédé comprenant la modification de la séquence de codage de la Figure 5 de telle manière que, dans la protéine codée par la séquence de codage, le résidu R36 de la protéine codée par la séquence de la Figure 5 est absent ou est remplacé par un résidu d'acide aminé différent.

8. Un procédé de production d'un polypeptide de p66^{shc} modifié, comprenant la modification d'une séquence d'acides nucléiques telle que décrite à la revendication 7, et l'expression de l'acide nucléique modifié dans une cellule hôte.

9. Un procédé in vitro d'interruption du trajet de transduction du signal de p66 ^{shc} dans une cellule comprenant la mise en contact de ladite cellule avec une molécule d'acide nucléique capable d'une hybridisation à l'acide nucléique codant p66^{shc} en empêchant ainsi ladite expression de p66^{shc}.

10. Un procédé in vitro d'augmentation de la résistance dans des cellules à des contraintes oxydatives comprenant l'étape consistant à interrompre le trajet de signalisation de p66^{shc}.

11. Un procédé in vitro selon la revendication 10 dans lequel ladite étape consistant à interrompre le p66^{shc} affecte la susceptibilité de p66^{shc} à une phosphorylation.

12. Un procédé in vitro selon la revendication 10 ou la revendication 11 dans lequel ladite étape consistant à interrompre le trajet de p66 ^{shc} amène un polypeptide de p66^{shc} mutant à être exprimé de telle manière que le S36 du résidu de sérine de la protéine codée par la séquence de la Figure 5 est absent ou est remplacé par un résidu d'acide aminé différent.

13. Un procédé in vitro selon la revendication 12, dans lequel le résidu correspondant à S36 est remplacé par de l'alanine.

14. Un procédé in vitro d'augmentation de la résistance des cellules à une contrainte oxydative comprenant l'étape consistant à interrompre le trajet de signalisation de p66^{shc} par mise en contact de la cellule avec un anticorps capable de se lier de manière spécifique au polypeptide de p66^{shc} de telle manière qu'est empêchée une liaison ultérieure de p66^{shc} à d'autres protéines.

15. Un procédé in vitro d'augmentation de la résistance des cellules à une contrainte oxydative comprenant l'étape consistant à interrompre le trajet de signalisation de p66^{shc} par mise en contact de ladite cellule avec un oligonucléotide anti-sensibilité capable d'une hybridisation à l'acide nucléique codant le polypeptide de p66^{shc}.

16. Utilisation d'un oligonucléotide anti-sensibilité capable d'une hybridisation spécifique à l'acide nucléique de p66^{shc} dans la préparation d'un médicament pour augmenter la résistance des cellules à une contrainte oxydative.

17. Utilisation selon la revendication 16 dans laquelle la séquence d'acides nucléiques de p66^{shc} est montrée à la Fig. 5.

18. Utilisation d'un anticorps capable d'une liaison spécifique à un polypeptide de p66^{shc} ou à un fragment de celui -ci dans la préparation d'un médicament pour augmenter la résistance des cellules à une contrainte oxydative.

19. Utilisation d'un vecteur comprenant l'acide nucléique codant le polypeptide de p66^{shc} dans la préparation d'un médicament pour augmenter la résistance à la formation de tumeur dans un tis.su, ledit vecteur étant capable d'incorporer ledit acide nucléique dans le génome des cellules du tissu.
